# EUROPEAN PATENT SPECIFICATION

(11) **EP 0 754 016 B1**
(45) Date of publication and mention of the grant of the patent: **09.07.2003**
(21) Application number: 95916920.2
(22) Date of filing: 03.04.1995
(51) Int. Cl.: A61F 2/06

(54) **SELF-EXPANDABLE STENT AND STENT-GRAFT**
SELBSTAUSDEHNBARER STENT UND STENT-GRAFT TRANSPLANTAT
EXTENSEUR ET GREFFE A EXTENSEUR AUTODEPLOYABLES

(30) Priority: 01.04.1994 US 222263; 01.04.1994 US 221815; 31.08.1994 US 299190; 08.09.1994 US 303060; 23.11.1994 US 344158; 21.12.1994 US 361793; 14.01.1995 US 374474; 28.03.1995 US 411441; 28.03.1995 US 411443; 28.03.1995 US 411452
(43) Date of publication of application: 22.01.1997
(60) Divisional application: 00101667.4
(73) Proprietor: Prograft Medical, Inc., Palo Alto, CA 94303 (US)
(72) Inventor: LAU, Lilip, Sunnyvale, CA 94087 (US); MARONEY, Charles, T., Portola Valley, CA 94028 (US); HARTIGAN, William, M., Fremont, CA 94537 (US); LAM, Sharon, San Jose, CA 95129 (US); MCCULLOUGH, Kimberly, A., Hayward, CA 94544 (US); RHEE, Woonza, Palo Alto, CA 94306 (US)
(74) Representative: Shanks, Andrew
(86) International application number: US9504000
(87) International publication number: WO95026695

(56) References cited:
- EP-A- 0 540 290
- EP-A- 0 556 850
- EP-A- 0 565 251
- WO-A-92/06734
- WO-A-93/13825
- WO-A-94/00179
- US-A- 4 994 071
- US-A- 5 100 429
- US-A- 5 122 154
- US-A- 5 133 732
- US-A- 5 195 984

## Description

### FIELD OF THE INVENTION

This invention is a medical device . The device is a foldable stent or stent-graft which may be delivered with (or on) a catheter or via surgical or other suitable techniques. The device is then expanded or unfolded. The expandable stent structure utilizes at least one torsional member generally aligned with the longitudinal axis of the stent. The stent has an undulating shape. It is helically deployed to form the generally cylindrical shape deployed as the stent. The structure is aligned to allow those undulating shapes in adjacent turns of the helix to be in phase. The adjacent undulating shapes are held in that phased relationship using a flexible linkage, often made of a polymeric material. The stent's configuration allows it to be folded or otherwise compressed to a very small diameter prior to deployment without changing the length of the stent. The stent is self-expanding, kink-resistant, easily bent along its longitudinal axis, does not change its length during that expansion, and is able to provide collapsible support for otherwise frangible graft material. The graft component cooperating with the stent is tubular and may be a biocompatible polymeric or collagenous material or combinations of the two which may, if desired, be reinforced with fibers.

The procedures for deploying stents or stent-grafts which have been folded, bound, or otherwise collapsed to significantly smaller diameters for insertion into a human or animal body may involve the use of an outer sleeve to maintain the stent or stent-graft at a reduced diameter or may involve a "slip-line" to hold and then to release the device.

### BACKGROUND OF THE INVENTION

With the advent of interventional radiology, the treatment or isolation of a variety of maladies in the body's conduits may be easily treated using stents and stent-grafts. For instance, this invention may be used to treat weakened, distorted, narrowed, or otherwise malformed vessels in the vascular, biliary, genito-urinary, gastrointestinal, and respiratory systems. Of special interest in the use of this invention is the treatment of vascular aneurysms or of arterial or venous vessel walls which have been thinned or thickened by disease. Much of this vascular treatment has traditionally been done via surgery, e.g., via the use of surgical bypassing with vascular grafts. Shortcomings of this procedure include the morbidity and mortality associated with surgery, long recovery times after surgery, and the high incidence of repeat intervention needed due to limitations of the graft or of the procedure. Vessels thickened by disease are currently sometimes treated less invasively with intraluminal stents that mechanically hold these vessels open either subsequent to or as an adjunct to a balloon angioplasty procedure. Shortcomings of current stents as used in the vascular system include the use of highly thrombogenic materials (stainless steels, tantalum, ELGILOY®) which are exposed to blocd, the general failure of these materials to attract and support functional endothelium, the irregular stent/vessel surface that causes unnatural blood flow patterns, and the mismatch of compliance and flexibility between the vessel and the stent.

Highly desirable is the use of less invasive intraluminal delivery and, when used in the vascular system, placement of a nonthrombogenic blood-carrying conduit having a smooth inner lumen which will endothelize.

A desirable graft material chosen for the inner layer of the inventive stent-graft is collagen-based and, although it will fold with ease, is otherwise fairly frangible or inelastic in that it has very little ability to stretch. Mounting a collagen tube on the outside of or as a part of a balloon-expandable stent will usually cause the tube to tear. Mounting such a tube on the inside of a balloon expandable stent will yield a torn irregular surface exposed to blood flow. Further, balloon expandable devices that rely upon plastic deformation of the stent to achieve a deployed shape are subject to abrupt closure as a result of trauma when the devices are placed in a vessel near the skin surface or across a joint or ligament. Those self-expanding stents which rely on the shortening of the stent upon radial expansion at deployment may cause vessel tearing problems similar to those observed with the use of balloon expandable devices. Obviously, stents which shorten during deployment are also subject to deployment placement inaccuracies.

The most desired variations of this invention involve a stent-graft which is self-expanding, which does not shorten upon delivery, which has excellent longitudinal flexibility, which has high radial compliance to the vessel lumen, and exposes the blood to a smooth, nonthrombogenic surface capable of supporting endothelium growth.

The inventive device may be delivered in a reduced diameter and expanded to maintain the patency of any conduit or lumen in the body, particularly those mentioned above. An area in which the inventive stent and stent graft is particularly beneficial is in the scaffolding of atherosclerotic lesions in the cardiovascular system to establish vessel patency, prevention of thrombosis, and the further prevention of re-stenosis after angioplasty. In contrast to many of the stents discussed below having metallic struts intruding into the blood flow in the vessel lumen which generate turbulence and create blood stasis points initiating thrombus formation, the smooth, continuous surface provided by the tubular collagen-based, polymer-based, or combination inner conduit of our invention provides a hemodynamically superior surface for blood flow.

The non-thrombogenic properties of an sPEG collagen surface results in a less thrombogenic device. Clinically, this allows a more moderate anti-coagulation regimen to be used. As a result, the rate of bleeding complications, a major drawback associated with stenting, may be reduced. The absence of gaps or holes in the graft structure between stent struts of our invention allows the tacking of both large and small flaps and tears in the vessel wall. These flaps disrupt blood flow and attract thrombus. The disruption of the natural anti-thrombotic covering of endothelium only worsens the condition. The collagen-based barrier we interpose between blood and a disrupted or injured portion of the vessel wall serves to mask injured intimal or medial layers from blood, thereby preventing thrombus formation and intimal proliferation which may lead to re-stenosis.

The presence of our inventive stent-graft acts as a mechanical barrier preventing tissue from proliferating into or impinging the lumen. The nature of the bioactivity of the collagen and the smoother flow characteristics at the blood-contacting surface are conducive to endothelial cell attachment and growth thereby assuring the long-term blood compatibility of the device.

Mechanically, our stent structure provides a good combination of radial strength and flexibility. The structure is also radially resilient. It can be completely crushed or flattened and yet spring open again once the obstructive loading is removed. This ability is important for use in exposed portions of the body around the peripheral vasculature or around joints. The stent-graft can sustain a crushing traumatic blow or compression from the bending of a joint and still return to the open configuration once the load is removed.

With regard to delivery, the self-expansion mechanism eliminates the need for a balloon catheter and the associated balloon rupture problems often associated with balloons. In addition, the absence of the bulk of the balloon allows a smaller delivery profile to be achieved. Unlike some other self-expanding stent designs, this stent-graft maintains a constant length throughout the expansion process. Thus, the stent-graft would not have some of the positioning problems associated with other many self-expanding stents. In treating longer lesions, our self-expanding design eliminates the need for special long balloons or repositioning of the balloon between inflations in order to expand the entire length of the stent.

When used as a conventional vascular graft or intraluminal graft, our collagen-based stent-grafts offer a number of advantages over existing technologies. Unlike expanded polytetrafluoroethylene (PTFE) grafts, the bare collagen-based material supports endothelial cell growth and is incorporated into the surrounding tissue. As an intraluminal graft, the device has several advantages. The wall thickness may be made thinner than tanned, reinforced biologic grafts. When placed inside the lumen of a vessel, a thin-walled graft results in a larger opening for blood flow resulting in improved hemodynamics. Lastly, when used as an intraluminal graft, there is no anastomosis site. Anastomosis sites are thought to be a common source of problems associated with graft failures.

The impermeability of the inventive stent-graft makes it suitable for shunting and thereby hydraulically isolating aneurysms. The expansile properties derived from the stent structure provide a secure anchor to the vessel wall. The stent reinforces frangible graft materials making up the tubular component thereby increasing the overall burst strength of the stent-graft.

Finally, the organic composition of the collagen-based materials which may be used in the inventive stent-graft provides an excellent vehicle for localized drug delivery. In addition, therapeutic compounds may be linked, conjugated, or otherwise more easily bound to the organic graft material (or to its substituents, such as PEG) than to the surface of a metallic structure. Localized drug delivery is desirable in preventing thrombosis or re-stenosis. Therapeutically effective doses may be administered to the target area without systemic concentrations being raised. This capability is of great benefit in reducing side-effects and complications associated with drug therapy.

Therapeutic agents may be delivered out of the collagen matrix by diffusion. Alternatively, these agents may be bound temporarily or permanently on the collagen surfaces. Different agents may be bound on the inner and outer surfaces to achieve different therapeutic ends. For example, a drug to minimize thrombus formation might be appropriate for the inside, blood-contacting surface, while a drug which would inhibit smooth muscle cell proliferation might be appropriate on the outer surface. Drugs can be chemically or physically bound to either the sPEG or the collagen molecules.

### Stents

The stents currently described in the open literature include a wide variety of different shapes.

Wallsten, U.S. Patent No. 4,655,771, suggests a vascular prosthesis for transluminal implantation which is made up of a flexible tubular body having a diameter that is varied by adjusting the axial separation of the two ends of the body relative to each other. In general, the body appears to be a woven device produced of various plastics or stainless steel.

U.S. Patent No. 4,760,849, to Kroph, shows the use of a ladder-shaped coil spring which additionally may be used as a filter in certain situations.

Porter, U.S. Patent No. 5,064,435, suggests a stent made up of two or more tubular stent segments which may be deployed together so to produce a single axial length by a provision of overlapping areas. This concept is to permit the use of segments of known length, which, when deployed, may be used together in overlapping fashion additively to provide a stent of significant length.

Quan-Gett, U.S. Patent No. 5,151,105, discloses an implantable, collapsible tubular sleeve apparently of an outer band and an inner spring used to maintain the sleeve in a deployed condition.

Wall, U.S. Patent No. 5,192,307, suggests a stent having a number of holes therein and which is expandable using an angioplasty balloon so to allow ratchet devices or ledges to hold the stent in an open position once it is deployed.

The following patents use wire as the stent material.

Gianturco, in U.S. Pat. Nos. 4,580,568 and 5,035,706, describes stents formed of stainless steel wire arranged in a closed zigzag pattern. The stents are compressible to a reduced diameter for insertion into and removal from a body passageway. The stents appear to be introduced into the selected sites by discharge of the collapsed zigzag wire configuration from the tip of a catheter.

U.S. Patent Nos. 4,830,003 and 5,104,404, to Wolff et al., shows a stent of a zigzag wire configuration very similar in overall impression to the Gianturco device. However, the stent is said to be self-expanding and therefore does not need the angioplasty balloon for its expansion.

Hillstead, U.S. Patent 4,856,516, suggests a stent for reinforcing vessel walls made from a single elongated wire. The stent produced is cylindrical and is made up of a series of rings which are, in turn, linked together by half-hitch junctions produced from the single elongated wire. It is not helically wound, nor is there a second linking member tending to link the helices together.

Wiktor, U.S. Patent Nos. 4,649,992, 4,886,062, 4,969,458, and 5,133,732, shows wire stent designs using variously a zigzag design or, in the case of Wiktor '458, a helix which winds back upon itself. Wiktor '062 suggests use of a wire component made of a low-memory metal such as copper, titanium or gold. These stents are to be implanted using a balloon and expanded radially for plastic deformation of the metal.

Wiktor '458 is similarly made of low-memory alloy and is to be plastically deformed upon its expansion on an angioplasty balloon.

Wiktor '732 Leaches the use of a longitudinal wire welded to each turn of the helically wound zig-zag wire which is said to prevent the longitudinal expansion of the stent during deployment. A further variation of the described stent includes a hook in each turn of the helix which loops over a turn in an adjacent turn. Neither variation includes a flexible linkage between adjacent helices.

WO93/13825, to Maeda et al, shows a tabular self-expanding stent as defined in the preamble of claim 1. This prior art stent is defined by an undulating member helically wrapped about a central longitudinal axis, the undulating member containing a pluraltity of undulations, and a flexible linkage weaved to extend through the undulations of adjacent turns to maintain turns of adjacent undulations in phase with one another. The undulations each have small loops or "eyes" at their apexes which are interconnected with the flexible linkage. Because of the teaching to connect the eyes of the apexes, the stent appears to be a design that axially expands during compression and may tear attached grafts because of the relative change in position of the arms of the zig-zag during compression of the stent.

MacGregor, U.S. Pat. No. 5,015,253, shows a tubular non-woven stent made up of a pair of helical members which appear to be wound using opposite "handedness". The stent helices desirably are joined or secured at the various points where they cross.

Pinchuk, in U.S. Pat. Nos. 5,019,090, 5,092,877, and 5,163,958, suggests a spring stent which appears to circumferentially and helically wind about as it is finally deployed except, perhaps, at the very end link of the stent. The Pinchuk '958 patent further suggests the use of a pyrolytic carbon layer on the surface of the stent to present a porous surface of improved antithrombogenic properties. The helices are not linked to each other, however, nor is there any suggestion that the helices be maintained in a specific relationship either as deployed or as kept in the catheter prior to deployment.

U.S. Patent No. 5,123,917, to Lee, suggests an expandable vascular graft having a flexible cylindrical inner tubing and a number of "scaffold members" which are expandable, ring-like, and provide circumferential rigidity to the graft. The scaffold members are deployed by deforming them beyond their plastic limit using, e.g., an angioplasty balloon.

Tower, in U.S. Pat. Nos. 5,161,547 and 5,217,483, shows a stent formed from a zig-zag wire wound around a mandrel in a cylindrical fashion. It is said to be made from "a soft platinum wire which has been fully annealed to remove as much spring memory as possible." A longitudinal wire is welded along the helically wound sections much in the same way as was the device of Wiktor.

There are a variety of disclosures in which super-elastic alloys such as nitinol are used in stents. See, U.S. Patent Nos. 4,503,569, to Dotter; 4,512,338, to Balko et al.; 4,990,155, to Wilkoff; 5,037,427, to Harada, et al.; 5,147,370, to MacNamara et al.; 5,211,658, to Clouse; and 5,221,261, to Termin et al. None of these references suggest a device having discrete, individual, energy-storing torsional members as are required by this invention.

Jervis, in U.S. Pat. Nos. 4,665,906 and 5,067,957, describes the use of shape memory alloys having stress-induced martensite properties in medical devices which are implantable or, at least, introduced into the human body.

### Stent-Grafts

A variety of stent-graft designs are shown in the following literature.

Perhaps the most widely known such device is shown in Ersek, U.S. Pat. No. 3,657,744. Ersek shows a system for deploying expandable, plastically deformable stents of metal mesh having an attached graft through the use of an expansion tool.

Palmaz describes a variety of expandable intraluminal vascular grafts in a sequence of patents: U.S. Patent Nos. 4,733,665; 4,739,762; 4,776,337; and 5,102,417. The Palmaz '665 patent suggests grafts (which also function as stents) that are expanded using angioplasty balloons. The grafts are variously a wire mesh tube or of a plurality of thin bars fixedly secured to each other. The devices are installed, e.g., using an angioplasty balloon and consequently are not seen to be self-expanding.

The Palmaz '762 and '337 patents appear to suggest the use of thin-walled, biologically inert materials on the outer periphery of the earlier-described stents.

Finally, the Palmaz '417 patent describes the use of multiple stent sections each flexibly connected to its neighbor.

Rhodes, U.S. Pat. No. 5,122,154, shows an expandable stent-graft made to be expanded using a balloon catheter. The stent is a sequence of ring-like members formed of links spaced apart along the graft. The graft is a sleeve of a material such as expanded polyfluorocarbon, e.g., GORETEX or IMPRAGRAFT.

Schatz, U.S. Pat. No. 5,195,984, shows an expandable intraluminal stent and graft related in concept to the Palmaz patents discussed above. Schatz discusses, in addition, the use of flexibly-connecting vascular grafts which contain several of the Palmaz stent rings to allow flexibility of the overall structure in following curving body lumen.

Cragg, "Percutaneous Femoropopliteal Graft Placement", Radiology, vol. 187, no. 3, pp. 643-648 (1993), shows a stent-graft of a self-expanding, nitinol, zig-zag, helically wound stent having a section of polytetrafluoroethylene tubing sewed to the interior of the stent.

Cragg (EP-A- 0556850) discloses an intraluminal stent made up of a continuous helix of zig-zag wire and having loops at each apex of the zig-zags. Those loops on the adjacent apexes are individually tied together to form diamond-shaped openings among the wires. The stent may be made of a metal such as nitinol (col. 3, lines 15-25 and col. 4, lines 42+) and may be associated with a "polytetrafluoroethylene (PTFE), dacron, or any other suitable biocompatible material". Those biocompatible materials may be inside the stent (col. 3, lines 52+) or outside the stent (col. 4, lines 6+). There is no suggestion that the ties are free to move away from the apexes of the zig-zags and that the zig-zag wire helix is aligned "in phase". The alignment of the wire and the way in which it is tied mandates that it expands in length as it is expanded from its compressed form.

### Grafts

As was noted above, the use of grafts in alleviating a variety of medical conditions is well known. Included in such known grafting designs and procedures are the following.

Medell, U.S. Patent No. 3,479,670, discloses a tubular prothesis adapted to be placed permanently in the human body. It is made of framework or support of a synthetic fibre such as DACRON (a registered trade mark) or TEFLON (a registered trade mark). The tube is said to be made more resistant to collapse by fusing a helix of a polypropylene monofilament to its exterior. The reinforced fabric tube is then coated with a layer of collagen or gelatin to render the tubing (to be used as an esophageal graft) impermeable to bacteria or fluids.

Sparks, in U.S. Patent Nos. 3,514,791, 3,625,198, 3,710,777, 3,866,247, and 3,866,609, teach procedures for the production of various graft structures using dies of suitable shape and a cloth reinforcing material within the die. The die and reinforcement are used to grow a graft structure using a patient's own tissues. The die is implanted within the human body for a period of time to allow the graft to be produced. The graft is in harvested and implanted in another site in the patient's body by a second surgical procedure.

Braun, in U.S. Patent No. 3,562,820, shows a biological prosthesis manufactured by applying onto a support of a biological tissue (such as serosa taken from cattle intestine) a collagen fiber paste. The procedure is repeated using multiple layers of biological tissue and collagen fiber paste until a multi-layer structure of the desired wall thicknesses is produced. The prosthesis is then dried and removed prior to use.

Dardik et al, U.S. Patent No. 3,974,526, shows a procedure for producing tubular prostheses for use in vascular reconstructive surgeries. The prosthesis is made from the umbilical cord of a newly born infant. It is washed with a solution of 1 percent hydrogen peroxide and rinsed with Ringer's lactate solution. It is then immersed in a hyaluronidase solution to dissolve the hyaluronic acid coating found in the umbilical cord. The vessels are then separated from the cord and their natural interior valving removed by use of a tapered mandrel. The vessels are then tanned with glutaraldehyde. A polyester mesh support is applied to the graft for added support and strength.

Whalen, U.S. Patent No. 4,130,904, shows a prosthetic blood conduit having two concentrically associated tubes with a helical spring between them. Curved sections in the tube walls help prevent kinking of the tube.

Ketharanathan, U.S. Patent No. 4,319,363, shows a procedure for producing a vascular prosthesis suitable for use as a surgical graft. The prosthesis is produced by implanting a rod or tube in a living host and allowing collagenous tissue to grow on the rod or tube in the form of coherent tubular wall. The collagenous implant is removed from the rod or tube and tanned in glutaraldehyde. The prosthesis is then ready for use.

Bell, U.S. Patent No. 4,546,500, teaches a method for making a vessel prosthesis by incorporating a contractile agent such as smooth muscle cells or platelets into a collagen lattice and contracting the lattice around a inner core. After the structure has set, additional layers are applied in a similar fashion. A plastic mesh sleeve is desirably sandwiched between the layers or imbedded within the structure to provide some measure of elasticity.

Hoffman Jr. et al, U.S. Patent No. 4,842,575, shows a collagen impregnated synthetic vascular graft. It is made of a synthetic graft substrate and a cross-linked collagen fibril. It is formed by depositing a aqueous slurry of collagen fibrils into the lumen of the graft and massaging the slurry into the pore structure of the substrate to assure intimate admixture in the interior. Repeated applications and massaging and drying is said further to reduce the porosity of the graft.

Alonoso, U.S. Patent No. 5,037,377, is similar in overall content to the Hoffman Jr. et al patent discussed above except that, in addition to collagen fibers, soluble collagen is introduced into the fabric. A suitable cross-linking agent such as glutaraldehyde is used to bond adjacent collagen fibers to each other.

Slepian et al, U.S. Patent No. 5,213,580, teaches a process described as "paving" or "stabilizing by sealing the interior surface of a body vessel or organ" by applying a biodegradable polymer such as a polycaprolactone. The polymer is made into a tubular substrate, placed in position, and patched into place.

Finally, there are known vascular grafts using collagenous tissue with reinforcing structure. For instance, Pinchuk, in U.S. Patent Nos. 4,629,458 and 4,798,606, suggests the use of collagen with some other type of fibrous structure supporting the collagen as a biograft. Similarly, Sinofsky et al., U.S. Pat. No. 5,100,429, suggests a partially-cured, collagen-based material used to form a graft within a blood vessel.

Kreamer, U.S. Pat. No. 4,740,207, suggests a intraluminal graft made of a semi-rigid resilient tube, open along a seam extending from one end to the other, which is expanded within the vessel and which resulting larger diameter is maintained by use of a ledge at the longitudinal seam for catching the opposite side of the seam on the expanded graft.

We have found that elasticity, or the ability of a material to return to its original shape after a deformation, can be maintained in smaller stents by the distribution of folding deformation throughout the structure. By incorporating hinges or hinge regions into the structure, the distribution of "localized" folding deformation is maximized. The hinge regions include torsion members allowing a significant portion of the folding displacement to be re-oriented parallel to the longitudinal axis of the stent-graft. The act of folding, crushing, or otherwise elastically deforming the stent creates a significant torsional component in the torsion members which component is parallel to that longitudinal axis. The hinges are positioned at least at each of the fold points around the circumference of the stent where folding is desired. The circumferentially oriented regions of the stents, which connect the torsion members, pivot about the torsion members, causing the torsion members to undergo a twisting deformation. In order to avoid exceeding the elastic limit of the material, the length of the torsion members is increased to lower the amount of twist per length or strain imposed. The orientation of the torsion members is such that their length does not increase the circumference of the device. None of the cited references suggest such a device.

### SUMMARY OF THE INVENTION

According to the present invention, there is provided a tubular self-expanding stent as defined in claim 1. Further features of the invention are defined in the dependent claims.

### BRIEF DESCRIPTION OF THE DRAWINGS

Figures 1A, 1B, 1C, and 1D are plan views of an unrolled stent form making up the helical variation of the invention.
Figure 2 is a side view of the inventive helical stent.
Figure 3 is a close-up of a portion of the inventive helical stent shown in Figure 2.
Figure 4 is an abstracted portion of an inventive helical stent and shows the result of torsion on a portion of that stent.
Figure 5 is a side view of the inventive helical stent showing a variation having flared ends.
Figures 6, 7 and 8 show plan views of an unrolled helical stent produced from flat stock.
Figure 9 shows a device for winding and heat treating a stent made according to the invention.
Figures 10 and 11 are close-ups of a portion of the inventive stent-graft showing multiple distributed attachment points between the stent and the graft.
Figure 12 shows a front quarter view of a stent graft of the type shown in Figures 10 and 11.
Figures 13 and 14 show end view cutaways of stent-grafts made according to the invention.
Figure 15 shows the placement of a continuous graft on a stent graft covering the entrance to a side branch.
Figures 16, 17, 18 and 19 show side-views of stent-grafts with non-continuous graft surfaces.
Figures 20A, 20C, and 20E show procedures for folding the stent-grafts made according to the invention. Figures 20B, 20D and 20F show the corresponding folded stent-grafts.
Figures 21A-21C show a schematic procedure for deploying the inventive stent-grafts using an external sleeve.
Figures 22A and 23A show front quarter views of folded stents or stent-grafts held in that folded position by a tether wire. Figures 22B, 22C, 23B, and 23C show end views of the folded stent and of the open stent shown respectively in Figures 22A and 23A.
Figures 24A-24C show a schematic procedure for deploying the inventive stent-grafts (as shown in Figures 22A-22C and 23A-23C) using a tether wire.
Figure 25 shows a close-up view of a stent fold line using a preferred sack knot in the slip line.
Figures 26 and 27 show front quarter views of folded stents or stent-grafts held in that folded position by a tether wire using a sack knot.

### DESCRIPTION OF THE EMBODIMENTS OF INVENTION

As was noted above, this invention is an expandable stent and a stent-graft.

The stent-graft may be a combination of the following: a thin-walled tube (or graft) generally coaxial with the stent and the expandable stent structure. The tubular graft may comprise a porous polymeric tube, e.g., of an expanded PTFE, having a collagenous material embedded in the pores of the tube. The graft material may optionally contain fibrous reinforcement material. The stent and the optional reinforcing fibers may be imbedded in the wall of the thin-walled tube. The expandable stent structure is a generally cylindrical body produced of a helically placed (wound or otherwise preformed) torsion member having an undulating or serpentine shape.

When the undulating torsion member is formed into the cylinder, the undulations are aligned so that they are "in phase" with each other. The undulations are linked, with a flexible linkage of a suitable metallic or polymeric material, to maintain the phased relationship of the undulations during compression and deployment and during bending of the stent. These stent configurations are exceptionally kink-resistant and flexible, particularly when flexed along the longitudinal axis of the stent.

When the stent is used in a reinforced stent-graft, that is to say: the stent is included into a thin-walled tube having reinforcing fibers, the fibers may be formed into a network, such as a tubular mesh. The stent-graft may be delivered percutaneously through the vasculature after having been folded to a reduced diameter. Once reaching the intended delivery site, it is expanded to form a lining on the vessel wall.

Central to one variation of the invention is the distributed attachment of the stent component to the graft component via, e.g., the bonding of the graft to the filament which may used to maintain the stent in its tubular shape to allow the stent to move locally with respect to the graft and maintain the open structure of the graft lumen.

A further variation of the invective includes stent-grafts which are have open areas to allow access between the inner lumen end outer surface through the stent structure.

### Stent Component

The materials typically used for vascular grafts, e.g., synthetic polymers or fabrics or collagen, usually do not have the stiffness or strength alone both to stay open against the radial inward loads found in those vessels and to prevent their slippage from the chosen deployment site. In order to provide the strength required, a radially rigid stent structure may be incorporated into the stent-graft. Our stent is constructed of a reasonably high strength material, i.e., one which is resistant to plastic deformation when stressed. The structure is typically from one of three sources:
1.) a wire form in which a wire is first formed into an undulating shape and the resulting undulating shape is helically wound to form a cylinder,
2.) an appropriate shape is formed from a flat stock and wound into a cylinder, and
3.) a length of tubing is formed into an appropriate shape.
These stent structures are typically oriented coaxially with the tubular graft component. The stent structures may be placed on the outer surface or the inner surface of the tubular member although the stent may be imbedded in the graft tubing wall for ease of integration with the tubing and to prevent the stent's exposure to bodily fluids, such as blood. It is desired that the stent structure have the strength and flexibility to tack the graft tubing firmly and conformally against the vessel wall. In order to minimize the wall thickness of the stent-graft, the stent material should have a high strength-to-volume ratio. The designs do not suffer from a tendency to twist (or helically unwind) or to shorten as the stent is deployed. As will be discussed below, materials suitable in these stents and meeting these criteria include various metals and some polymers.

A stent or stent-graft, whether delivered percutaneously or via a body orifice must expand from the reduced diameter necessary for delivery to a larger deployed diameter. The diameters of these devices obviously vary with the size of the body lumen into which they are placed. For instance, the stents of this invention may range in size from 2.0mm in diameter (for vascular neurological applications) to 30mm in diameter (for placement in the aorta). A range of about 2.0mm to 6.5mm (perhaps to 10.0mm) is believed to be desirable. Typically, expansion ratios of 2:1 or more are required. These stents are capable of expansion ratios of up to 5:1 for larger diameter stents. Typical expansion ratios for use with the stents and stent-grafts of the invention typically are in the range of about 2:1 to about 4:1 although the invention is not so limited. The thickness of the stent materials obviously varies with the size (or diameter) of the stent and the ultimate required yield strength of the folded stent. These values are further dependent upon the selected materials of construction. Wire used in these variations are typically of stronger alloys, e.g., nitinol® and stronger spring stainless steels, and have diameters of about 0.002 to 0.005 inches (0.00508 to 0.0127 cm). For the larger stents, the appropriate diameter for the stent wire may be somewhat larger, e.g., 0.005 to 0.020 inches (0.0127 to 0.0508 cm). For flat stock metallic stents, thicknesses of about 0.002 to 0.005 inches (0.00508 to 0.0127 cm) is usually sufficient. For the larger stents, the appropriate thickness for the stent flat stock may be somewhat thicker, e.g., 0.005 to 0.020 inches (0.0127 to 0.0508 cm).

The stent-graft is fabricated in the expanded configuration. In order to reduce its diameter for delivery the stent-graft would be folded along its length, similar to the way in which a PCTA balloon would be folded. It is desirable, when using super-elastic alloys which also have temperature-memory characteristics, to reduce the diameter of the stent at a temperature below the transition-temperature of the alloys. Often the phase of the alloy at the lower temperature is somewhat more workable and easily formed. For instance, at nitinol® martensitic temperatures, the alloy material provides minimal resistance to folding and tends to maintain the folded configuration. The temperature of deployment is desirably above the transition temperature to allow use of the super-elastic properties of the alloy.

Thus, a preferred method for folding the stent-graft (when super-elastic alloys are used) comprises the steps of chilling the stent-graft to the martensitic temperature of the alloy, folding the stent-graft to the desired reduced diameter configuration and constraining the stent-graft in that folded configuration. The device is then allowed to warm to the austenitic temperature of the alloy (e.g., when the austenitic temperature is at or below room temperature) or above. This warming can be done, for example, before or after it is packaged. In use, the folded stent-graft is delivered to the treatment site and the constraint removed so that it can return to its original configuration to serve its intended purpose. Alternatively, heat from the body or another source coupled to the alloy material can be used to trigger the shape memory of the material. In that case, the alloy is selected to have an austenitic temperature above room temperature. Heat from the body or another source is used to heat the alloy material to its austenitic temperature as the device is delivered to the selected site so that upon release of the constraint, the device returns to its original configuration.

### Helical stents

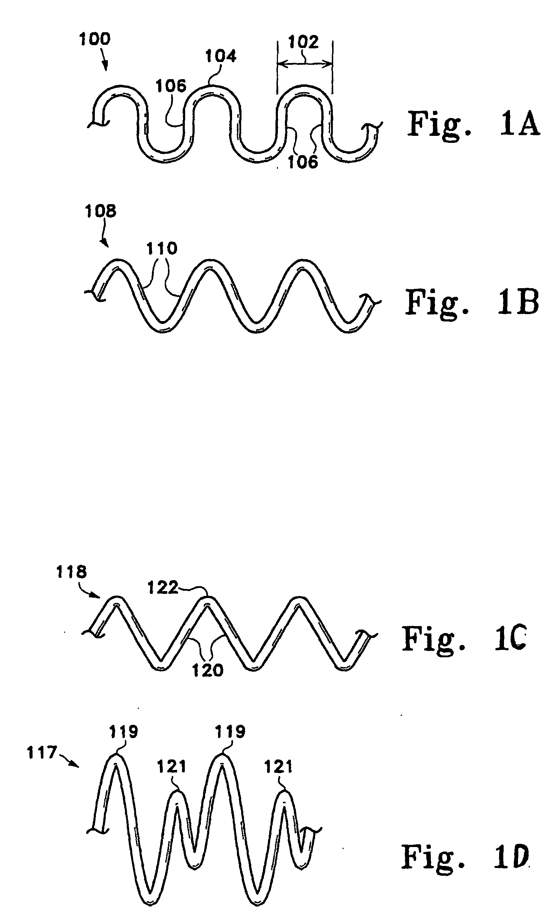

As a generic explanation of the mechanical theory of the helical variation of the inventive stent, reference is made to Figures 1A, 1B, 1C, 1D, 2, 3, and 4. Figure 1A is a plan view of an isolated section of the inventive stent device and is intended both to identify a variation of the invention and to provide conventions for naming the components of the torsion member (100). Figure 1A shows, in plan view, an undulating torsion member (100) formed from a wire stock into a U-shape. A torsion pair (102) is made up of an end member (104) and two adjacent torsion lengthe (106). Typically, then, each torsion length (106) will be a component to each of its adjacent torsion pairs (102). The U-shaped torsion pair (102) may be characterized, by the fact that the adjacent torsion lengths are generally parallel to each other prior to formation into the stent.

The stente of this invention use undulating torsion members which are "open" or "unconfined" at their apex or end member (104). By "open" or "unconfined" we mean that the apex or end member (104) does not have any means in that apex which would tend to inhibit the movement of the flexible linkage (discussed below) down between the arms or torsion lengths (106) of the torsion pair (102).

Figure 1B shows another variation of the invention having a sinusoidal shaped torsion member (108). In this variation, the adjacent torsion lengths (110) are not parallel and the wire forms an approximate sine shape before being formed into a cylinder.

Figure 1C shows another variation of the invention having a V-shaped torsion member (118). In this variation, the adjacent torsion lengths (120) form a relatively sharp angle at the torsion end (122) shape before being formed into a cylinder.

Figure 1D shows a variation of the invention in which adjacent torsion members on the stent (117) have differing amplitude.

The configurations shown in Figs 1A-1D are exceptionally kink-resistant and flexible when flexed along the longitudinal axis of the stent.

As ultimately deployed, a section of the torsion member found on one of Figures 1A - 1D is helically wound about a form of an appropriate size so that the end members (e.g., 104 in Figure 1A) are centered between the end members of the torsion member on an adjacent turn of the helix. This is said to be "in phase". "Out of phase" would have been the instance in which the adjacent members meet directly, i.e., end member-to-end member. Once so aligned, the phasic relationship may be stabilized by weaving a flexible linkage through the end members from one turn of the helix to the next.

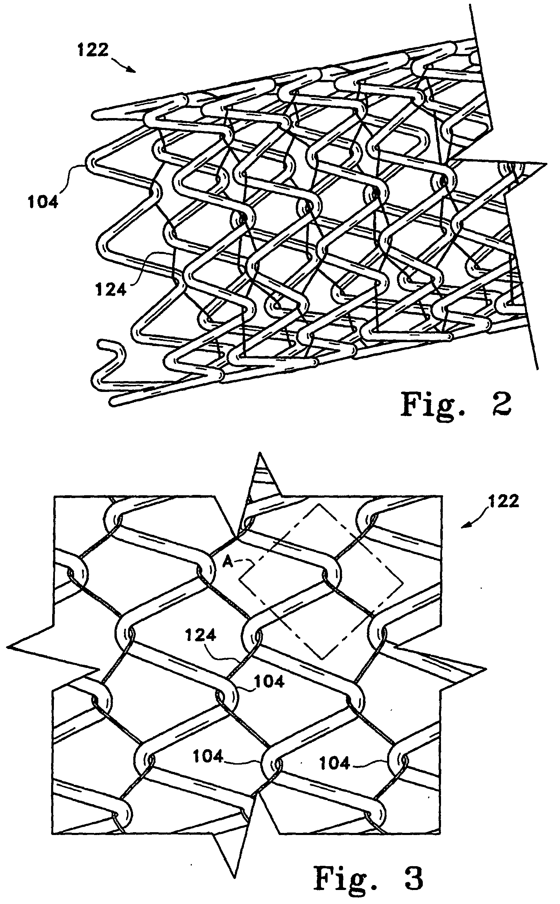

Figure 2 shows a side view of a typical stent (122) made according to this invention including the phased relationship of the helical turns of the stent and the flexible linkage (124). Figure 3 shows a close-up of the Figure 2 stent and depicts the phased relationship (within box A) and shows in detail a typical way in which the flexible linkage (124) is looped through the various end members (104) to maintain the phased relationship. It may be noted that the flexible linkage (124) is free to move away from the apex at the end members (104) without constraint.

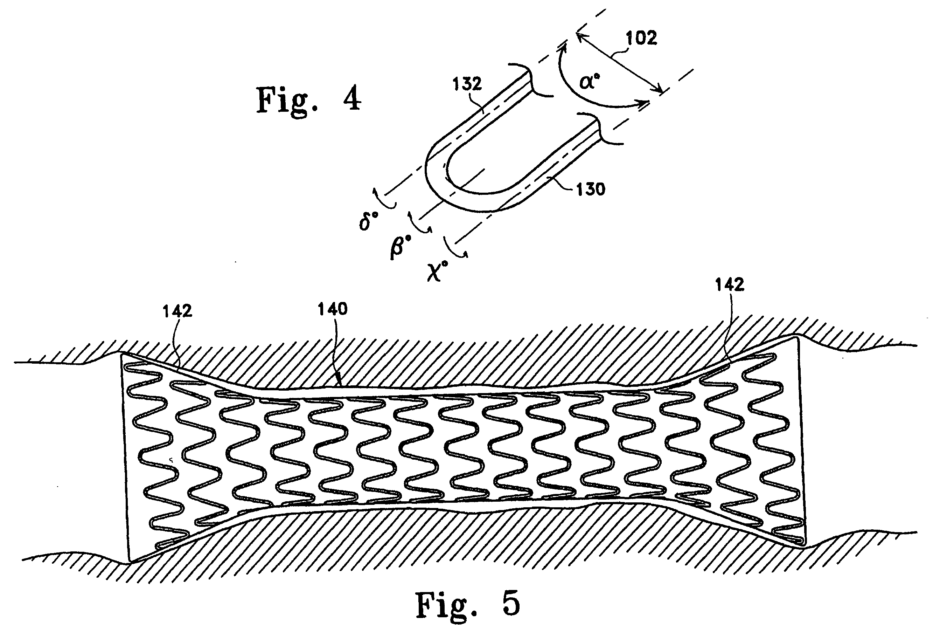

The stent may be folded in some fashion (as will be discussed below) for deployment. During the step of folding, the stent undergoes a transformation. Figure 4 shows an isolated torsion pair (102). When the torsion pair (102) undergoes a flexing in the amount of α°, the end member will flex some amount β°, torsion length (130) will undertake a twist of γ°, and torsion length (132) will undertake a twist opposite of that found in torsion length (130) in the amount of δ°. The amounts of angular torsion found in the torsion lengths (130 and 132) will not necessarily be equal because the torsion lengths are not necessarily at the same angle to the longitudinal axis of the stent. Nevertheless, the sum of β°+γ°+δ° will equal α°. When a value of α° is chosen, as by selection of the shape and size of the stent upon folding, the values of the other three angles ( β°,γ°,δ°) are chosen by virtue of selection of number of torsion pairs around the stent, size and physical characteristics of the wire, and length of the torsion lengths (130 and 132). Each of the noted angles must not be so large as to exceed the values at which the chosen material of construction plastically deforms at the chosen value of α°.

To further explain the invention: it should understood that the torsion pair (102) undergoes a significant of flexing as the stent is folded or compressed in some fashion. The flexing provides a twist to the torsion lengths (130 and 132), a significant portion of which is generally parallel to the longitudinal axis of the stent. It is this significant imposed longitudinal torsion which forms an important concept of the inventive stent.

As noted elsewhere, in one very desirable variation of the inventive stent, as deployed in Figures 2 and 3, the stent is folded longitudinally and is delivered through the lumen of the catheter in such a way that it is self-restoring once it has been introduced to the selected body lumen site. This stated desire is not to rule out the use of the inventive stent or stent-graft with a balloon or expander or other shape-restoring tool if so desired, but the design of the stent is meant to eliminate the need for (or, at least to minimize the need for) such expanding tools.

With that preliminary background in place, it should be apparent that a simple tube of metal will undergo plastic deformation when sufficient force is applied radially to the outside of the tube. The amount of force needed to cause that plastic deformation will depend on a wide variety of factors, e.g., the type of metal utilized in the tube, the width of the tube, the circumference of the tube, the thickness of the material making up the band, etc. The act of attempting to fold a tube along its centered axis in such a way to allow it to pass through a lumen having the same or smaller diameter and yet maintain the axis of the folded stent parallel to the axis of the lumen invites plastic deformation in and of the stent.

The inventive helical stent uses concepts which can be thought of as widely distributing and storing the force necessary to fold the tubular stent into a configuration which will fit through a diameter smaller than its relaxed outside diameter without inducing plastic deformation of the constituent metal or plastic and yet allowing those distributed forces to expand the stent upon deployment.

Once the concept of distributing the folding or compression stresses both into a bending component (as typified by angle β° in Figure 4) and to twisting components (as typified by angle γ° and δ° in Figure 4), and determining the overall size of a desired stent, determination of the optimum materials as well as the sizes of the various integral components making up the stent becomes straightforward. Specifically, the diameter and length of torsion lengths (130 and 132) and end sector (104), the number of torsion pairs (102) around the stent may then be determined.

Figure 5 shows, in side view, a variation of the inventive stent (140) made from wire having flares (142) at one or both ends. The flaring provides a secure anchoring of the stent or stent-graft (140) against the vessel wall. This prevents the implant from migrating downstream. In addition, the flaring provides a tight seal against the vessel so that the blood is channelled through the lumen rather than outside the graft. The undulating structure may vary in spacing to allow the helix turns to maintain its phased relationship between turns of the helix and to conform to the discussion just above. A flexible linkage between the contiguous helical turns may also be applied to at least a portion of the helices.

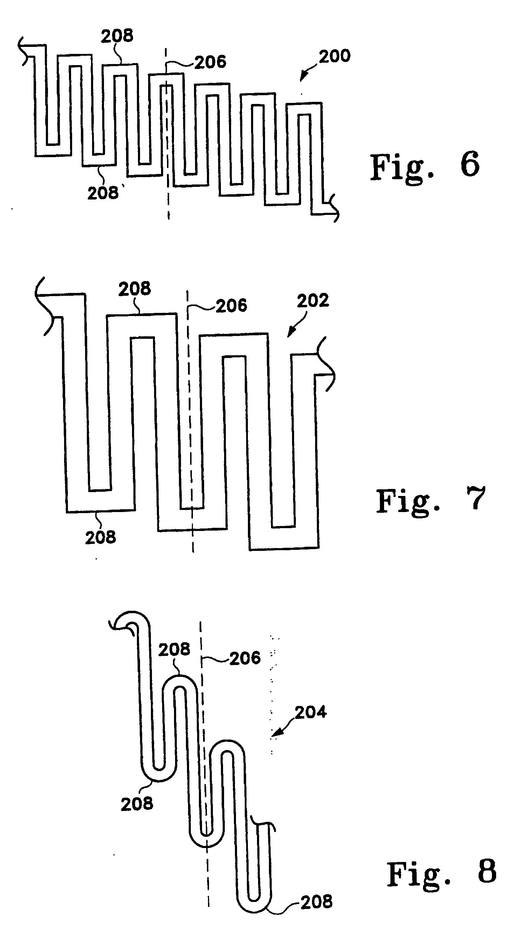

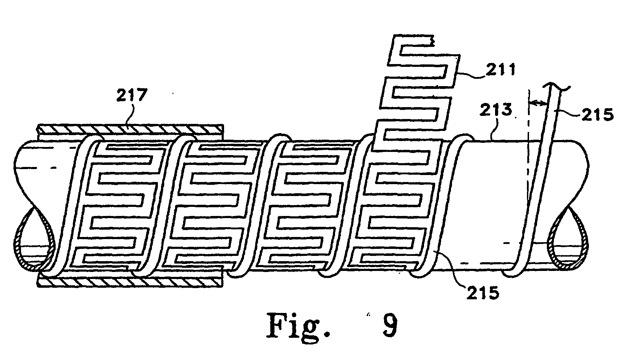

The helical stent structure may also be made by forming a desired structural pattern out of a flat sheet. The sheet may then be rolled to form a tube. Figures 6, 7, and 8 show plan views of torsion members (respectively 200, 202, and 204) which may be then rolled about an axis (206) to form a cylinder.

It should be clear that a variety of materials variously metallic, super elastic alloys, and preferably nitinol, are suitable for use in these stents. Primary requirements of the materials are that they be suitably springy even when fashioned into very thin sheets or small diameter wires. Various stainless steels which have been physically, chemically, and otherwise treated to produce high springiness are suitable as are other metal alloys such as cobalt chrome alloys (e.g., ELGILOY), platinum/tungsten alloys, and especially the nickel-titanium alloys generically known as "nitinol".

Nitinol is especially preferred because of its "super-elastic" or "pseudo-elastic" shape recovery properties, i.e., the ability to withstand a significant amount of bending and flexing and yet return to its original form without deformation. These metals are characterized by their ability to be transformed from an austenitic crystal structure to a stress-induced martensitic structure at certain temperatures, and to return elastically to the austenitic shape when the stress is released. These alternating crystalline structures provide the alloy with its super-elastic properties. These alloys are well known but are described in U.S. Pat. Nos. 3,174,851, 3,351,463, and 3,753,700. Typically, nitinol® will be nominally 50.6% (±0.2%) Ni with the remainder Ti. Commercially available nitinol® materials usually will be sequentially mixed, cast, formed, and separately cold-worked to 30-40%, annealed, and stretched. Nominal ultimate yield strength values for commercial nitinol® are in the range of 206,8 kPa (30 psi) and for Young's modulus are about 70 GPa(700 kBar).

The '700 patent describes an alloy containing a higher iron content and consequently has a higher modulus than the Ni-Ti alloys. Nitinol® is further suitable because it has a relatively high strength to volume ratio. This allows the torsion members to be shorter than for less elastic metals. The flexibility of the stent-graft is largely dictated by the length of the torsion member components in the stent structural component. The shorter the pitch of the device, the more flexible the stent-graft structure can be made. Materials other than nitinol® are suitable. Spring tempered stainless steels and cobalt-chromium alloys such as ELGILOY® are also suitable as are a wide variety of other known "super-elastic" alloys.

Although nitinol® is preferred in this service because of its physical properties and its significant history in implantable medical devices, we also consider it to be suitable for use as a stent because of its overall suitability with magnetic resonance imaging (MRI) technology. Many other alloys, particularly those based on iron, are an anathema to the practice of MRI causing exceptionally poor images in the region of the alloy implant. Nitinol® does not cause such problems.

Other materials suitable as the stent include certain polymeric materials, particularly engineering plastics such as thermotropic liquid crystal polymers ("LCP's"). These polymers are high molecular weight materials which can exist in a so-called "liquid crystalline state" where the material has some of the properties of a liquid (in that it can flow) but retains the long range molecular order of a crystal. The term "thermotropic" refers to the class of LCP's which are formed by temperature adjustment. LCP's may be prepared from monomers such as p,p'-dihydrexy-polynuclear-aromatics or dicarboxy-polynuclear-aromatics. The LCP's are easily formed and retain the necessary interpolymer attraction at room temperature to act *as* high strength plastic artifacts as are needed as a foldable stent. They are particularly suitable when augmented or filled with fibers such as those of the metals or alloys discussed below. It is to be noted that the fibers need not be linear but may have some preforming such as corrugations which add to the physical torsion enhancing abilities of the composite.

Figure 9 shows a method for producing a stent . In this procedure, a preformed strip (211) (preferably nitinol®) is rolled onto a mandrel (213) having a channel defined by a thread (215). The pitch of the strip (211) is selected using the criteria discussed above. The pitch angle (215) shown is about 20° but is not critical to the operation of the invention. Once the strip (211) is wound onto the mandrel (213) and the assembly is introduced into the outer sleeve (217), the strip (now in the shape of the stent) may then be annealed (or at least "heat set") to assist in maintaining the resulting stent in a useful shape. For use in stents of the sizes mentioned elsewhere, we have found that nitinol® devices may be heat treated for five minutes or less at temperatures of 500°C without significant lessening of the superelastic properties. Said another way, heating super-elastic alloys must be carried out with some care so as not to destroy or significantly lessen the super-elastic properties.

The flexible linkage between adjacent turns of the helix (124 in Figs. 2 and 3) may be of any appropriate filamentary material which is blood compatible or biocompatible and sufficiently flexible to allow the stent to flex and not deform the stent upon folding. Although the linkage may be a single or multiple strand wire (platinum, platinum/tungsten, gold, palladium, tantalum, stainless steel, etc.), much preferred is the use of polymeric biocompatible filaments. Synthetic polymers such as polyethylene, polypropylene, polyurethane, polyglycolic acid, polyesters, polyamides, their mixtures, blends, copolymers, mixtures, blends and copolymers are suitable; preferred of this class are polyesters such as polyethylene terephthalate including DACRON® and MYLAR® and polyaramids such as KEVLAR®, polyfluorocarbons such as polytetrafluoroethylene with and without copolymerized hexafluoropropylene (TEFLON® or GORETEX®), and porous or nonporous polyurethanes. Natural materials or materials based on natural sources such as collagen are especially preferred is this service.

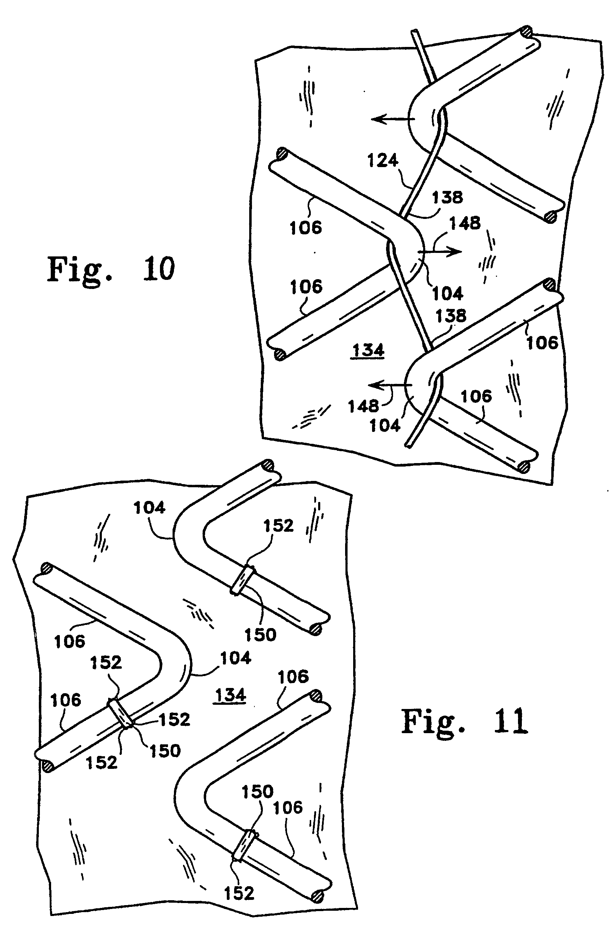

Figure 10 shows a magnified portion of a stent-graft (viewed from the outside of the stent-graft) incorporating a stent such as is shown in Figures 2 and 3 and depicts a method for distributively attaching the stent to the graft component. Specifically, end member or apex (104) is flanked by side lengths (106) and is looped therethrough by a filament (124). The graft component (134) is seen in the background. The filament (124) adheres to the graft (134) at the locations of contact (138) between the filament (124) and the graft component (134). It should be apparent that the apexes (104) are free to move in the direction shown by arrows (148) when the stent-graft is flexed. This shows the ability of the various apexes to move longitudinally with respect to each other and yet retain the graft component (134) reasonably snug against the inner surface of the stent and thereby prevent kinking of that graft component (134).

Figure 11 shows a close-up of a section of a stent-graft according to the invention that is similar to the stent-graft portion shown in Figure 10. Although no filament (124 in Figure 10) is shown in the variation in Figure 11, a filament (124) as shown in Figure 11 is used in conjunction with loops (150). The stent is attached to the graft using loops (150) or eyelets on the stent. Again this shows a manner of distributively attaching the stent to the graft component (134). Again, end member or apex (104) is flanked by side lengths (106). The graft component (134) is seen in the background. The loops (150) may be of a material which adheres to the graft component (134) at the junctions shown at (152). It is also contemplated that the filament (124) may be of material which is either adherent to (such as a melt-miscible thermoplastic polymer) or not adherent to (such as a metal or thermoset polymer) the graft component (134) when used with the loops (152).

The scope of materials for the filament (124), graft component (134), and loops (152) will be discussed in detail below.

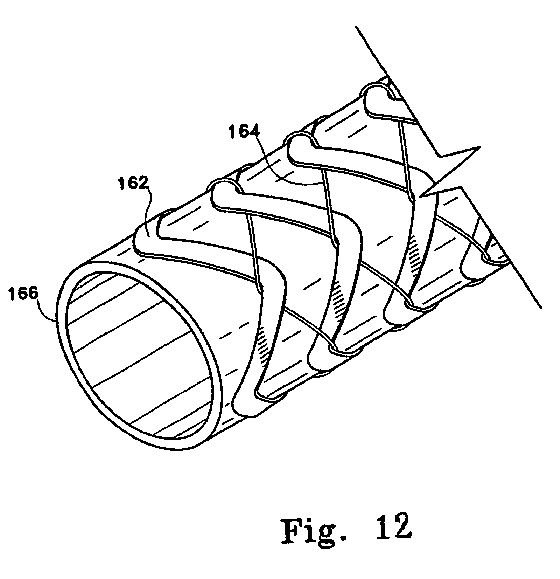

The stent support structure may also be made by forming a desired structural pattern out of a flat sheet. The formed sheet may then be rolled to form a tube, as is shown in Figure 12.

The flexible linkage (164) may then be included to preserve the diameter of the stent. The graft component (166) is shown on the inner surface of the stent. Loops may be used as was described above. The graft may be attached to the loops or filament in the manner discussed above.

### Tubular Graft Component

The tubular graft component or member of the stent-graft may be made up of any material which is suitable for use as a graft in the chosen body lumen. Many graft materials are known, particularly known are vascular graft materials. For instance, natural material may be introduced onto the inner surface of the stent and fastened into place. Synthetic polymers such as polyethylene, polypropylene, polyurethane, polyglycolic acid, polyesters, polyamides, their mixtures, blends, copolymers, mixtures, blends and copolymers are suitable; preferred of this class are polyesters such as polyethylene terephthalate including DACRON® and MYLAR® and polyaramids such as KEVLAR®, polyfluorocarbons such as polytetrafluoroethylene with and without copolymerized hexafluoropropylene (TEFLON® or GORETEX®), and porous or nonporous polyurethanes.

Especially preferred in this invention are the expanded fluorocarbon polymers (especially PTFE) materials described in British. Pat. Nos. 1,355,373, 1,506,432, or 1,506,432 or in U.S. Pat. Nos. 3,953,566, 4,187,390, or 5,276,276.

Included in the class of preferred expanded fluoropolymers are polytetrafluoroethylene (PTFE), fluorinated ethylene propylene (FEP), copolymers of tetrafluoroethylene (TFE) and per fluoro(propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is expanded PTFE.

Highly preferred materials are certain collagen-based materials of COLLAGEN CORPORATION of Palo Alto, California. The graft may adhere to or partially encapsulate or be cast about the stent when appropriate materials such as castable polyurethane or collagen-based materials are employed. When the stent-graft is produced in such a way that the openings in the stent contain graft material (as by casting), then we refer to such a stent-graft as an "integral stent-graft".

One very desirable variation of the invention involves the combination of a porous polymeric tubing member of one of the materials mentioned elsewhere herein, but most preferably of the expanded polyfluorocarbon polymers mentioned above, and a collagenous material imbedded into those pores. Specifically, the most preferred polymer is an expanded PTFE having an internodal distance of between 45 and 120 microns, more preferably between 60 and 90 microns. The preferred collagen material is that described below. This combination allows the polymeric tubing to provide structural reinforcement both for the overall stent and for the embedded collagen. The collagenous material still provides the benefits of endothelization mentioned elsewhere. The thickness of the graft need not be greater than the thickness of the polymeric tubing by itself, although it may be. The combination of synthetic polymeric tubing and collagenous material provides enhanced adhesion between the collagenous material and the stent and resistance to radial dimension changes (e.g., ballooning) which may be a problem when collagen is used alone.

A highly preferred collagen-based material is described in U.S. Pat. No. 5,162,430, to Rhee et al, or as described below. Collagen is easily formed into thin-walled tubes which are limp, compliant, flexible, uniform, and have smooth surfaces. The tubing walls may have a hydrated thickness of 0.001 to 0.020 inches (0.00254 to 0.0508 cm) [or to 0.100 inches (0.254 cm) in some cases] for efficacy. Other thicknesses may be used if specific goals are to be achieved. In a stent-graft, the collagen tube acts as an intravascular blood conduit to line the interior surface of the blood vessel. It isolates the lined segment of the vessel from direct contact with blood flow, tacks any tears or dissections, helps reinforce the vessel wall to protect against or isolate aneurysms, and provides a smooth, relatively thin, conformal surface for the blood flow. Of most importance (at least from the perspective of the most preferred aspects of our invention), specific collagenous materials, such as the collagen-hydrophilic polymer conjugate described in U.S. Pat. No. 5,162,430 and as described below, are very desirable as the tubular component in this stent-graft in that they form non-thrombogenic surfaces which will support the growth of endothelium.

The preferred collagen composition used in this invention is a pharmaceutically acceptable non-immunogenic composition formed by covalently binding atelopeptide collagen to pharmaceutically pure, synthetic, hydrophilic polymers via specific types of chemical bonds to provide collagen/polymer conjugates. Any type of collagen may be used including extracted and purified collagen including atelopeptide collagen which can be type I, type II or type III collagen. The collagen may be extracted from various sources such as bovine hide and human placenta and may be fibrillar or non-fibrillar. The synthetic hydrophilic polymer may be polyethylene glycol and derivatives thereof having a weight average molecular weight over a range of from about 100 to about 20,000. The compositions may incorporate other components such as biologically active materials. The collagen-polymer conjugates generally contain large amounts of water when formed. The extruded materials may be dehydrated, resulting in a reasonably flexible material which can be readily stored.

The term "collagen" as used herein refers to all forms of collagen, including those which have been extracted, processed or otherwise modified. Preferred collagens are non-immunogenic and, if extracted from animals, are treated to remove the immunogenic telopeptide regions ("atelopeptide collagen"), are soluble, and may be in the fibrillar or non-fibrillar form. Type I collagen is best suited to most applications involving bone or cartilage repair. However, other forms of collagen are also useful in the practice of the invention, and are not excluded from consideration here. Collagen crosslinked using heat, radiation, or chemical agents such as glutaraldehyde may be conjugated with polymers as described herein to form particularly rigid compositions. Collagen crosslinked using glutaraldehyde or other (nonpolymer) linking agents is referred to herein as "GAX", while collagen cross-linked using heat and/or radiation is termed "HRX." Collagen used in connection with the preferred embodiments of the invention is in a pharmaceutically pure form such that it can be incorporated into a body, human or otherwise, for the intended purpose.

The term "synthetic hydrophilic polymer" as used herein refers to a synthetic polymer having an average molecular weight and composition which renders the polymer essentially water-soluble. Preferred polymers are highly pure or are purified to a highly pure state such that the polymer is, or is treated to become, pharmaceutically pure. Most hydrophilic polymers can be rendered water-soluble by incorporating a sufficient number of oxygen (or, less frequently, nitrogen) atoms available for forming hydrogen bonds in aqueous solutions. Preferred polymers are hydrophilic but not soluble. Preferred hydrophilic polymers used herein include polyethylene glycol, polyoxyethylene, polymethylene glycol, polytrimethylene glycols, polyvinyl-pyrrolidones, and derivatives thereof. The polymers can be linear or multiply branched and will not be substantially crosslinked. Other suitable polymers include polyoxyethylene-polyoxypropylene block polymers and copolymers. Polyoxyethylene-polyoxypropylene block polymers having an ethylene diamine nucleus (and thus having four ends) are also available and may be used in the practice of the invention. Naturally occurring and/or biologically active polymers such as proteins, starch, cellulose, heparin, and the like are not generally desirable in this definition although they may be used. All suitable polymers will be non-toxic, non-inflammatory and non-immunogenic when used to form the desired composition, and will preferably be essentially non-degradable in vivo over a period of at least several months. The hydrophilic polymer may increase the hydrophilicity of the collagen, but does not render it water-soluble. Presently preferred hydrophilic polymers are mono-, di-, and multi-functional polyethylene glycols (PEG). Monofunctional PEG has only one reactive hydroxy group, while difunctional PEG has reactive groups at each end. Monofunctional PEG preferably has a weight average molecular weight between about 100 and about 15,000, more preferably between about 200 and about 8,000, and most preferably about 4,000. Difunctional PEG preferably has a molecular weight of about 400 to about 40,000, more preferably about 3,000 to about 10,000. Multi-functional PEG preferably has a molecular weight between about 3,000 and 100,000.

PEG can be rendered monofunctional by forming an alkylene ether at one end. The alkylene ether may be any suitable alkoxy radical having 1-6 carbon atoms, for example, methoxy, ethoxy, propoxy, 2-propoxy, butoxy, hexyloxy, and the like. Methoxy is presently preferred. Difunctional PEG is provided by allowing a reactive hydroxy group at each end of the linear molecule. The reactive groups are preferably at the ends of the polymer, but may be provided along the length thereof.

The term "chemically conjugated" as used herein means attached through a covalent chemical bond. In the practice of the invention, a synthetic hydrophilic polymer and collagen may be chemically conjugated by using a linking radical, so that the polymer and collagen are each bound to the radical, but not directly to each other. The term "collagen-polymer" refers to collagen chemically conjugated to a synthetic hydrophilic polymer, within the meaning of this invention. Thus, "collagen-PEG" (or "PEG-collagen) denotes a composition within the most preferred aspect of the invention wherein collagen is chemically conjugated to PEG. "Collagen-dPEG" refers to collagen chemically conjugated to difunctional PEG, wherein the collagen molecules are typically crosslinked. "Crosslinked collagen" refers to collagen in which collagen molecules are linked by covalent bonds with polyfunctional (including difunctional) polymers. Terms such as "GAX-dPEG" and "HRX-dPEG" indicate collagen crosslinked by both a difunctional hydrophilic polymer and a crosslinking agent such as glutaraldehyde or heat. The polymer may be "chemically conjugated" to the collagen by means of a number of different types of chemical linkages. For example, the conjugation can be via an ester or urethane linkage, but is more preferably by means of an ether linkage. An ether linkage is preferred in that it can be formed without the use of toxic chemicals and is not readily susceptible to hydrolysis in vivo.

Those of ordinary skill in the art will appreciate that synthetic polymers such as polyethylene glycol cannot be prepared practically to have exact molecular weights, and that the term "molecular weight" as used herein refers to the weight average molecular weight of a number of molecules in any given sample, as commonly used in the art. Thus, a sample of PEG 2,000 might contain a statistical mixture of polymer molecules ranging in weight from, for example, 1,500 to 2,500 daltons with one molecule differing slightly from the next over a range. Specification of a range of molecular weight indicates that the average molecular weight may be any value between the limits specified, and may include molecules outside those limits. Thus, a molecular weight range of about 800 to about 20,000 indicates an average molecular weight of at least about 800, ranging up to about 20 kDa.

The term "available lysine residue" as used herein refers to lysine side chains exposed on the outer surface of collagen molecules, which are positioned in a manner to allow reaction with activated PEG. The number of available lysine residues may be determined by reaction with sodium 2,4,6-trinitrobenzenesulfonate (TNBS).

The term "growth factor" is used to describe biologically active molecules and active peptides (which may be naturally occurring or synthetic) which aid in healing or regrowth of normal tissue. The function of growth factors is two-fold: 1) they can incite local cells to produce new collagen or tissue, or 2) they can attract cells to the site in need of correction. As such, growth factors may serve to encourage "biological anchoring" of the collagen graft implant within the host tissue. As previously described, the growth factors may either be admixed with the collagen-polymer conjugate or chemically coupled to the conjugate. For example, one may incorporate growth factors such as epidermal growth factor (EGF), transforming growth factor (TGF) alpha, TGF_{β} (including any combination of TGF_{β}s), TGF_{β1}, TGF_{β2}, platelet derived growth factor (PDGF-AA, PDGF-AB, PDGF-BB), acidic fibroblast growth factor (FGF), basic FGF, connective tissue activating peptides (CTAP), β-thrombo-globulin, insulin-like growth factors, erythropoietin (EPO), nerve growth factor (NGF), bone morphogenic protein (BMP), osteogenic factors, and the like. Incorporation of growth factors can facilitate regrowth when the tubes are used in the treatment of defective or damaged channels. Furthermore, one may chemically link the growth factors to the collagen-polymer composition by employing a suitable amount of multi-functional polymer molecules during synthesis. The growth factors may then be attached to the free polymer ends by the same method used to attach PEG to collagen, or by any other suitable method. By tethering growth factors to the outer and/or inner surface of the graft material, the amount of grafts needed to carry out effective treatment is substantially reduced. Tubes which incorporate growth factors may provide effective controlled-release drug delivery. By varying the chemical linkage between the collagen and the synthetic polymer, it is possible to vary the effect with respect to the release of the biologic. For example, when an "ester" linkage is used, the linkage is more easily broken under physiological conditions, allowing for sustained release of the growth factor from the matrix. However, when an "ether" linkage is used, the bonds are not easily broken and the growth factor will remain in place for longer periods of time with its active sites exposed providing a biological effect on the natural substrate for the active site of the protein. It is possible to include a mixture of conjugates with different linkages so as to obtain variations in the effect with respect to the release of the biologic, e.g., the sustained release effect can be modified to obtain the desired rate of release.

The terms "effective amount" or "amount effective to treat" refer to the amount of composition required in order to obtain the effect desired. Thus, a "tissue growth-promoting amount" of a composition containing a growth factor refers to the amount of growth factor needed in order to stimulate tissue growth to a detectable degree. Tissue, in this context, includes connective tissue, bone, cartilage, epidermis and dermis, blood, and other tissues with particular emphasis on tissues which form channels such as veins, arteries, intestines and the like. The actual amount which is determined to be an effective amount will vary depending on factors such as the size, condition, sex, and age of the patient, the type of tissue or channel, the effect desired and type of growth factor, and can be more readily determined by the caregiver.

The term "sufficient amount" as used herein is applied to the amount of carrier used in combination with the collagen-polymer conjugates used in forming the tubes of the invention. A sufficient amount is that amount which, when mixed with the conjugate, renders it in the physical form desired, for example, extrudable tubes, extrudable cylinders having any desired cross-section, and so forth. Extrudable formulations may include an amount of a carrier sufficient to render the composition smoothly extrudable without significant need to interrupt the extrusion process. The amount of the carrier can be varied and adjusted depending on the size and shape and thickness of the wall of the tube being extruded. Such adjustments will be apparent to those skilled in the art upon reading this disclosure.

### Conjugates

To form the most desired collagen-conjugates used in the inventive stent-grafts, collagen must be chemically bound to a synthetic hydrophilic polymer. This can be carried out in a variety of ways. In accordance with the preferred method, the synthetic hydrophilic polymer is activated and then reacted with the collagen. Alternatively, the hydroxyl or amino groups present on the collagen can be activated and the activated groups will react with the polymer to form the conjugate. In accordance with a less preferred method, a linking group with activated hydroxyl or amino groups thereon can be combined with the polymer and collagen in a manner so as to concurrently react with both the polymer and collagen forming the conjugate. Other methods of forming the conjugates will become apparent to those skilled in the art upon reading this disclosure. Since the conjugates of the invention are to be used in the human body it is important that all of the components, including the polymer, collagen, and linking group, if used form a conjugate that is unlikely to be rejected by the body. Accordingly, toxic and/or immunoreactive components are not preferred as starting materials. Some preferred starting materials and methods of forming conjugates are described further below.

Although different hydrophilic synthetic polymers can be used in connection with forming the conjugate, such polymers must be biocompatible, relatively insoluble, but hydrophilic and is preferably one or more forms of polyethylene glycol (PEG), due to its known biocompatibility. Various forms of PEG are extensively used in the modification of biologically active molecules because PEG can be formulated to have a wide range of solubilities and because it lacks toxicity, antigenicity, immunogenicity, and does not typically interfere with the enzymatic activities and/or conformations of peptides. Further, PEG is generally non-biodegradable and is easily excreted from most living organisms including humans.

The first step in forming the collagen-polymer conjugates generally involves the functionalization of the PEG molecule. Various functionalized polyethylene glycols have been used effectively in fields such as protein modification (see Abuchowski et al., Enzymes as Drugs, John Wiley & Sons: New York, NY (1981) pp. 367-383; and Dreborg et al., Crit. Rev. Therap. Drug Carrier Syst. (1990) 6:315 ), peptide chemistry (see Mutter et al., The Peptides, Academic: New York, NY 2:285-332; and Zalipsky et al., Int. J. Peptide Protein Res. (1987) 30:740 ), and the synthesis of polymeric drugs (see Zalipsky et al., Eur. Polym. J. (1983) 19:1177; and Ouchi et al., J. Macromol. Sci. -Chem. (1987) A24:1011 ). Various types of conjugates formed by the binding of polyethylene glycol with specific pharmaceutically active proteins have been disclosed and found to have useful medical applications in part due to the stability of such conjugates with respect to proteolytic digestion, reduced immunogenicity and longer half-lives within living organisms.

One form of polyethylene glycol which has been found to be particularly useful is monomethoxy-polyethylene glycol (mPEG), which can be activated by the addition of a compound such as cyanuric chloride, then coupled to a protein (see Abuchowski et al., J. Biol. Chem. (1977) 252:3578 ). Although such methods of activating polyethylene glycol can be used in connection with the present invention, they are not particularly desirable in that the cyanuric chloride is relatively toxic and must be completely removed from any resulting product in order to provide a pharmaceutically acceptable composition.

Activated forms of PEG can be made from reactants which can be purchased commercially. One form of activated PEG which has been found to be particularly useful in connection with the present invention is mPEG-succinate-N-hydroxysuccinimide ester (SS-PEG) (see Abuchowski et al., Cancer Biochem. Biphys. (1984) 7:175 ). Activated forms of PEG such as SS-PEG react with the proteins under relatively mild conditions and produce conjugates without destroying the specific biological activity and specificity of the protein attached to the PEG. However, when such activated PEGs are reacted with proteins, they react and form linkages by means of ester bonds. Although ester linkages can be used in connection with the present invention, they are not particularly preferred in that they undergo hydrolysis when subjected to physiological conditions over extended periods of time (see Dreborg et al., Crit. Rev. Therap. Drug Carrier Svst. (1990) 6:315; and Ulbrich et al., J. Makromol. Chem. (1986) 187:1131 ).

It is possible to link PEG to proteins via urethane linkages, thereby providing a more stable attachment which is more resistant to hydrolytic digestion than the ester linkages (see Zalipsky et al., Polymeric Drug and Drug Delivery Systems, Chapter 10, "Succinimidyl Carbonates of Polyethylene Glycol" (1991) to disclose the chemistry involved in linking various forms of PEG to specific biologically active proteins). The stability of urethane linkages has been demonstrated under physiological conditions (see Veronese et al., Appl. Biochem. Biotechnol. (1985) 11:141; and Larwood et al., J. Labelled Compounds Radiopharm. (1984) 21:603 ). Another means of attaching the PEG to a protein can be by means of a carbamate linkage (see Beauchamp et al., Anal. Biochem. (1983) 131:25; and Berger et al., Blood (1988) 71:1641 ). The carbamate linkage is created by the use of carbonyldiimidazole-activated PEG. Although such linkages have advantages, the reactions are relatively slow and may take 2 to 3 days to complete.

The various means of activating PEG described above and publications cited in connection with the activation means are described in connection with linking the PEG to specific biologically active proteins and not collagen. However, the present invention now discloses that such activated PEG compounds can be used in connection with the formation of collagen-PEG conjugates. Such conjugates provide a range of improved characteristics and as such can be used to form the various compositions used in forming the tubes of the present invention. [Polymeric Drug and Drug Delivery Systems, Chapter 10, "Succinimidyl Carbonates of Polyethylene Glycol" (1991) to disclose the chemistry involved in linking various forms of PEG to specific biologically active proteins.]

As indicated above, the conjugates used in forming the grafts may be prepared by covalently binding a variety of different types of synthetic hydrophilic polymers to collagen. However, because the final product or conjugate obtained must have a number of required characteristics such as being extrudable from a nozzle, biocompatible and non-immunogenic, it has been found useful to use polyethylene glycol as the synthetic hydrophilic polymer. The polyethylene glycol must be modified in order to provide activated groups on one or preferably both ends of the molecule so that covalent binding can occur between the PEG and the collagen. Some specific functionalized forms of PEG are shown structurally below, as are the products obtained by reacting these functionalized forms of PEG with collagen.

The first functionalized PEG is difunctionalized PEG succinimidyl glutarate, referred to herein as (SG-PEG). The structural formula of this molecule and the reaction product obtained by reacting it with collagen is shown in Formula 1.

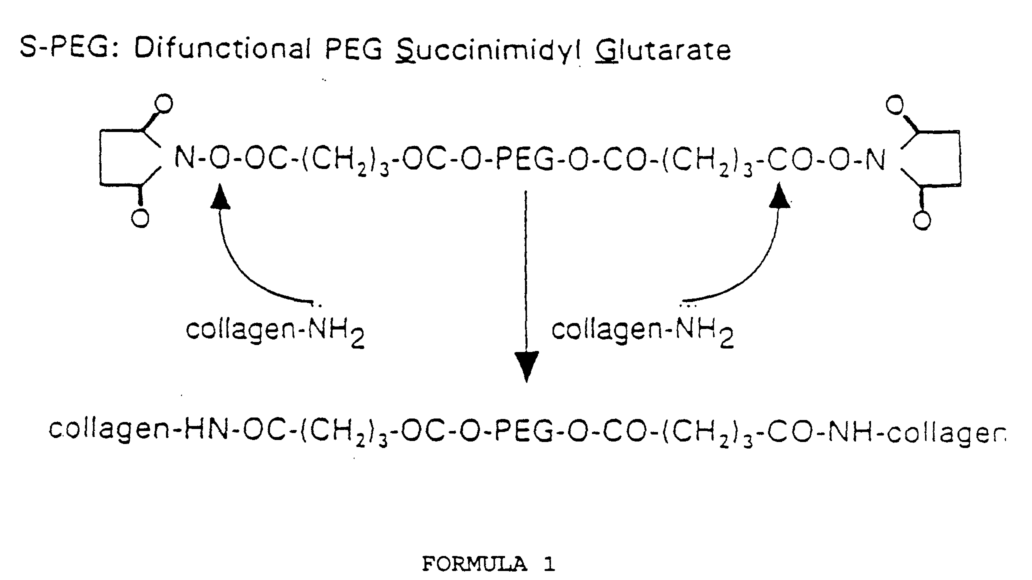

Another difunctionally activated form of PEG is referred to as PEG succinimidyl (S-PEG). The structural formula for this compound and the reaction product obtained by reacting it with collagen is shown in Formula 2. In a general structural formula for the compound of Formula 2, the subscript 3 is replaced with an "n." In the embodiment shown in Formula 1, n=3, in that there are three repeating CH₂ groups on each side of the PEG. The structure in Formula 2 results in a conjugate which includes an "ether" linkage which is not subject to hydrolysis. This is distinct from the first conjugate shown in Formula 1, wherein an ester linkage is provided. The ester linkage is subject to hydrolysis under physiological conditions.

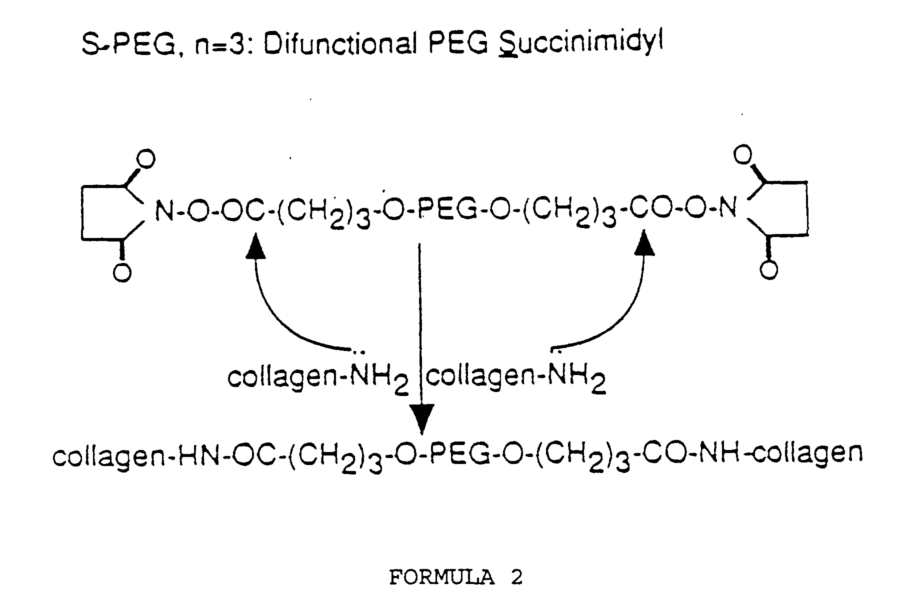

Yet another derivatized form of polyethylene glycol, wherein n=2 is shown in Formula 3, as is the conjugate formed by reacting the derivatized PEG with collagen. 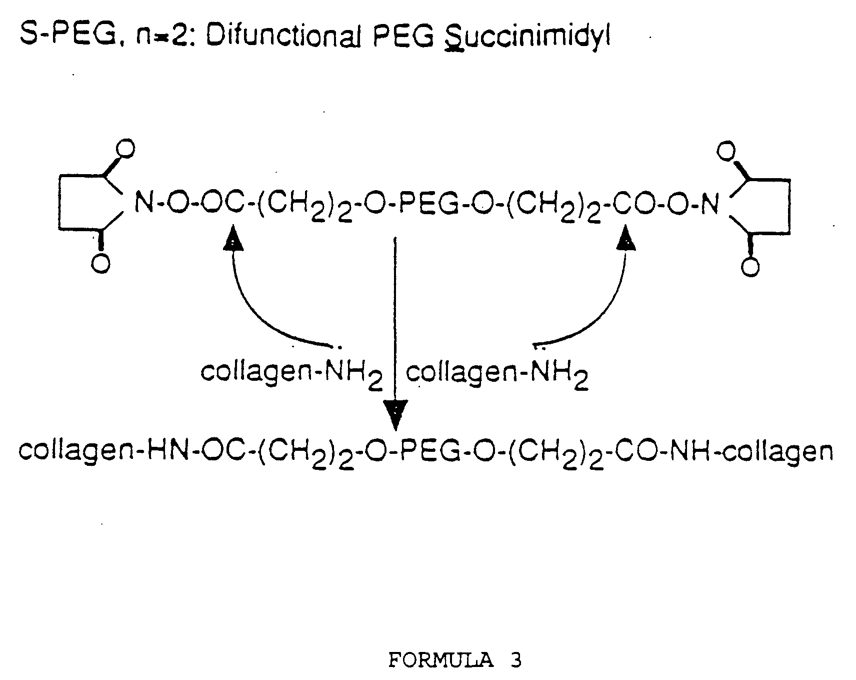 Another preferred embodiment of the invention similar to the compounds of Formula 2 and Formula 3, is provided when n=1. The structural formula and resulting conjugate are shown in Formula 4. It is noted that the conjugate includes both an ether and a peptide linkage. These linkages are stable under physiological conditions. 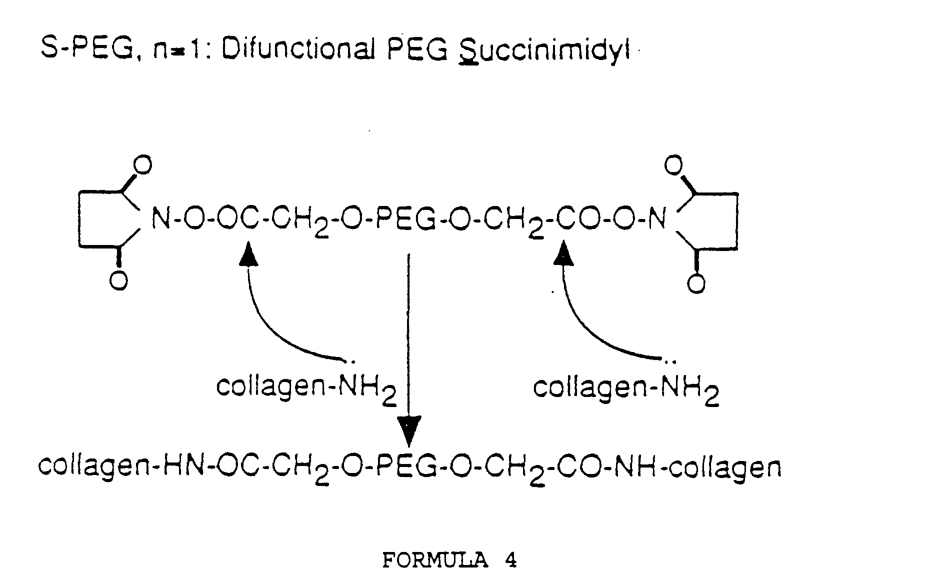 Yet another derivatized form of PEG is provided when n=0. The difunctionalized form is referred to as PEG succinimidyl carbonate (SC-PEG). The structural formula of this compound and the conjugate formed by reacting SC-PEG with collagen is shown in Formula 5. Although this conjugate includes a urethane linkage, the conjugate has been found not to have a high degree of stability under physiological conditions. The instability can be a desirable characteristic when the tubes are used in a situation where it is desirable that they dissolve over time.

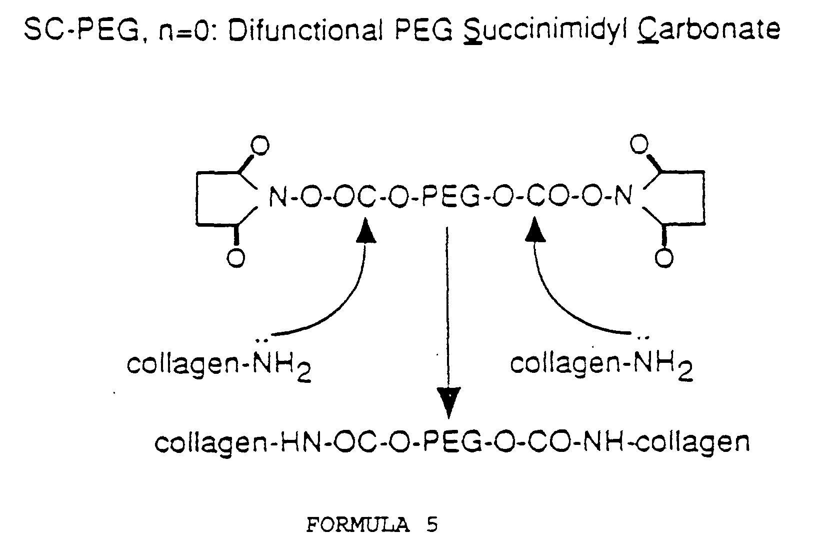

All of the derivatives depicted in Formulas 1-5 involve the inclusion of the succinimidyl group. However, different activating groups can be attached to one or both ends of the PEG. For example, the PEG can be derivatized to form difunctional PEG propionaldehyde (A-PEG), which is shown in Formula 6, as is the conjugate formed by the reaction of A-PEG with collagen.

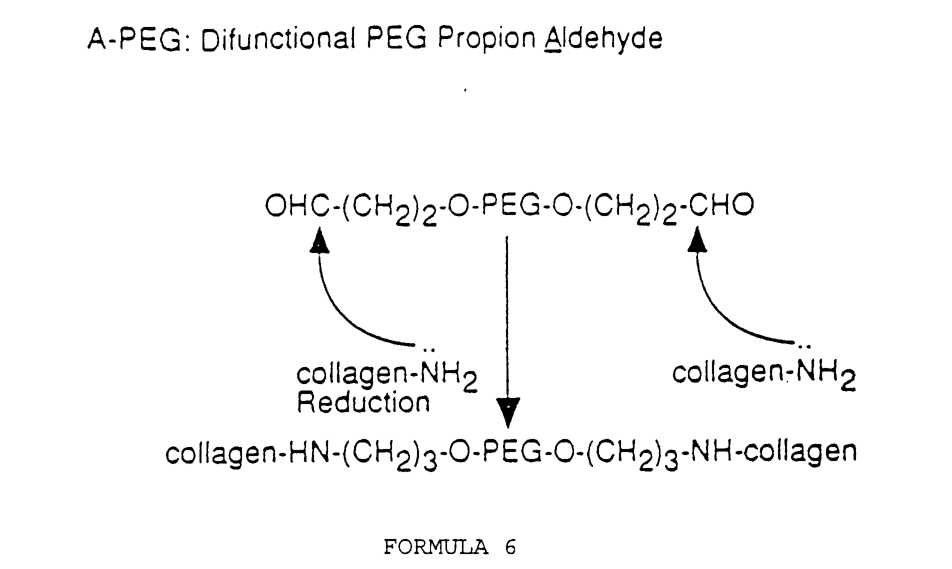

Yet another functionalized form of polyethylene glycol is difunctional PEG glycidyl ether (E-PEG), which is shown in Formula 7, as

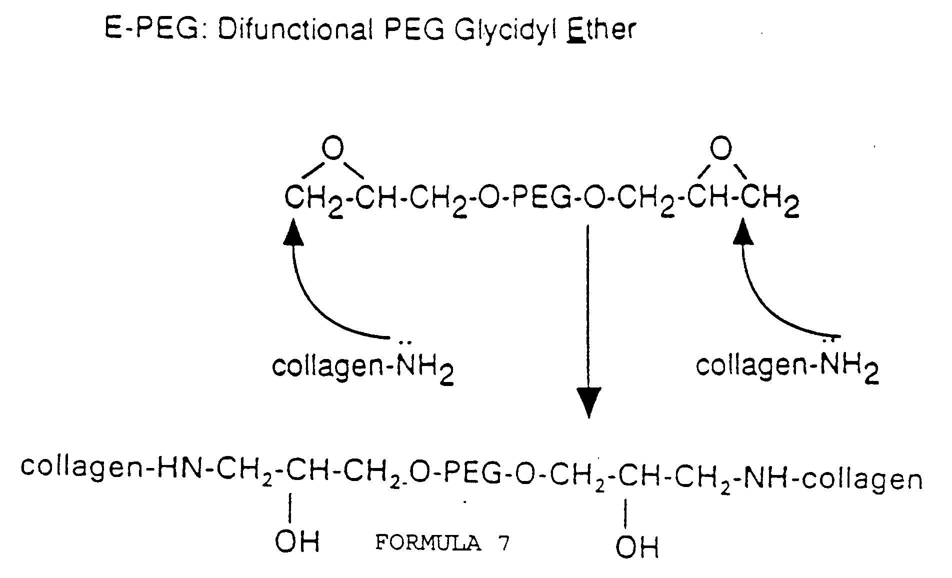

The conjugates formed using the functionalized forms of PEG vary depending on the functionalized form of PEG which is used in the reaction. Furthermore, the final product can be varied with respect to its characteristics by changing the molecular weight of the PEG. In general, the stability of the conjugate is improved by eliminating any ester linkages between the PEG and the collagen and including ether and/or urethane linkages. These stable linkages are generally used to form tubes to replace or augment a channel as may be done with a stent-graft. When the grafts are used as a temporary repair unit for a damaged channel, it may be desirable to include the weaker ester linkages so that the linkages are gradually broken by hydrolysis under physiological conditions, breaking apart the tube as it may be replaced by host tissue, or as it degrades, and releasing a component held therein, such as a growth factor. By varying the chemical structure of the linkage, the rate of sustained release can be varied.

Suitable collagens include all types of pharmaceutically useful collagen, preferably types I, II and III. Collagens may be soluble (for example, commercially available Vitrogen® 100 collagen-in-solution), and may or may not have the telopeptide regions. Preferably, the collagen will be reconstituted fibrillar atelopeptide collagen, for example Zyderm® collagen implant (ZCI) or atelopeptide collagen in solution (CIS). Various forms of collagen are available commercially, or may be prepared by the processes described in, for example, U.S. Pat. Nos. 3,949,073; 4,488,911; 4,424,208; 4,582,640; 4,642,117; 4,557,764; and 4,689,399. Fibrillar, atelopeptide, reconstituted collagen is preferred in order to form tubes used for the repair or augmentation of channels.

Compositions used in forming the graft used with the stent of the invention comprise collagen chemically conjugated to a selected synthetic hydrophilic polymer or polymers. Collagen contains a number of available amino and hydroxy groups which may be used to bind the synthetic hydrophilic polymer. The polymer may be bound using a "linking group", as the native hydroxy or amino groups in collagen and in the polymer frequently require activation before they can be linked. For example, one may employ compounds such as dicarboxylic anhydrides (e.g., glutaric or succinic anhydride) to form a polymer derivative (e.g., succinate), which may then be activated by esterification with a convenient leaving group, for example, N-hydroxy-succinimide, N,N'-disuccinimidyl oxalate, N,N'-disuccinimidyl carbonate, and the like. See also Davis, U.S. Pat. No. 4,179,337 for additional linking groups. Presently preferred dicarboxylic anhydrides that are used to form polymer-glutarate compositions include glutaric anhydride, adipic anhydride, 1,8-naphthalene dicarboxylic anhydride, and 1,4,5,8-naphthalenetetracarboxylic dianhydride. The polymer thus activated is then allowed to react with the collagen, forming a collagen-polymer composition used to make the grafts.

In one highly desirable embodiment having ester linkages, a pharmaceutically pure form of monomethylpoly-ethylene glycol (mPEG) (mw 5,000) is reacted with glutaric anhydride (pure form) to create mPEG glutarate. The glutarate derivative is then reacted with N-hydroxy-succinimide to form a succinimidyl monomethylpolyethylene glycol glutarate. The succinimidyl ester (mPEG*, denoting the activated PEG intermediate) is then capable of reacting with free amino groups present on collagen (lysine residues) to form a collagen-PEG conjugate wherein one end of the PEG molecule is free or nonbound. Other polymers may be substituted for the monomethyl PEG, as described above. Similarly, the coupling reaction may be carried out using any known method for derivatizing proteins and synthetic polymers. The number of available lysines conjugated may vary from a single residue to 100% of the lysines, preferably 10-50%, and more preferably 20-30%. The number of reactive lysine residues may be determined by standard methods, for example by reaction with TNBS.

The resulting product is a smooth, pliable, rubbery mass having a shiny appearance. It may be wetted, but is not water-soluble. It may be formulated as a suspension at any convenient concentration, preferably about 30-65 mg/mL, and may be extruded through a nozzle to form a tube. The consistency of the formulation may be adjusted by varying the amount of liquid used.

### Production of a Stent-Graft comprising collagen

One method of constructing a collagen-containing stent-graft is to first construct the stent and then to mold or cast the collagen tubular component about the stent.

The stent structure and any fiber reinforcement may be molded into the wall of the collagen tube. A mold for such a structure desirably is a simple annular space between two cylinders having room in the annular space for placement of the stent and would have a longitudinal axis slightly longer than the length of the stent-graft to be produced. The stent and fiber tubing is centered in the annular space and then the remaining space filled with collagen. If sPEG cross-linked collagen is used as the matrix material, the sPEG and collagen are mixed and introduced into the mold and allowed to cure. After curing, the mold is separated and the inventive fiber reinforced collagen tube with a stent structure produced.

Another method of producing a composite stent-graft is to attach a porous polymeric tubing to the stent in the manner mentioned elsewhere herein, e.g., by loop or attachment to the flexible linkage, and then to add the collagenous material to the pores in the tubing in the manner mentioned above.

### Stent-Graft

The tubular component, whether collagen-based or not, may also be reinforced using a network of small diameter fibers. The fibers may be random, braided, knitted, or woven. The fibers may be imbedded in the tubular component, may be placed in a separate layer coaxial with the tubular component, or may be used in a combination of the two.

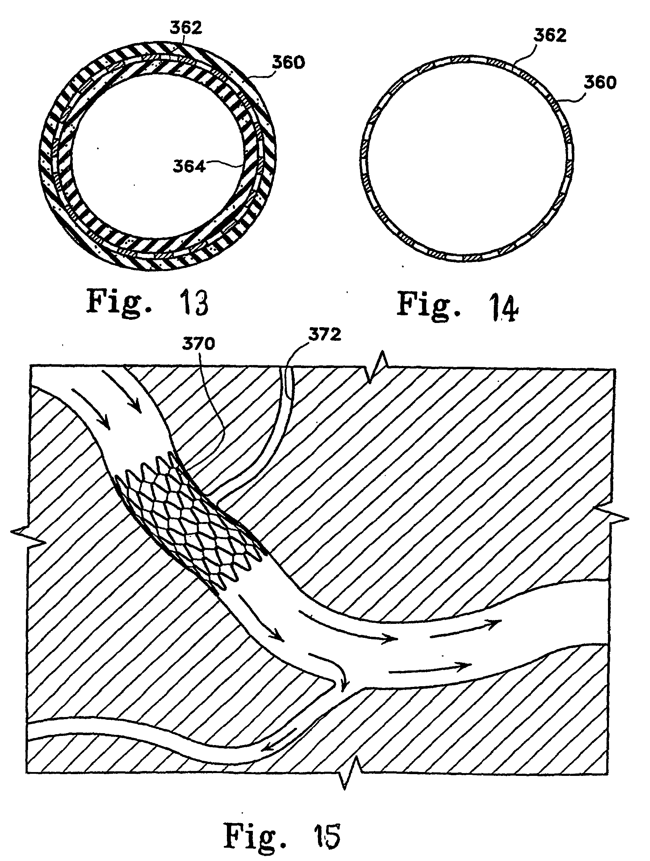

Figure 13 shows an end view, cross-section of the configuration in which the stent (360) forms the outermost layer, a fibrous layer (362) coaxial to and inside the stent (360), and the tubular component (364) of, e.g., collagen as the innermost layer.

Particularly desirable is the variation shown in Figure 14 in which the fibrous material is mixed with or imbedded into the tubular layer (364) and cast or injected around the stent (360). This fibrous material may extend for the length of the device or may be shorter. The fibers may be wound or placed in any reasonable orientation within the device. Alternatively, randomly oriented short segments of fibers may also be imbedded in the wall of the tubing. The fiber may be any suitable fibrous blood-compatible material including polyesters such as DACRON®, polyamides such as NYLON®, KEVLAR®, polyglycolic acids, polylactic acids, polyethylene, polypropylene, silk or other strong flexible fiber which are not detrimentally affected in the medical service in which this device is placed. Specifically, polypropylene and the like will not be dissolved in blood but polyglycolic acid will dissolve. Each are suitable but work in different ways.

In addition, one or more radio-opaque metallic fibers, such as gold, platinum, platinum-tungsten, palladium, platinum-iridium, rhodium, tantalum, or alloys or composites of these metals may be incorporated into the multi-strand reinforcement network to allow fluoroscopic visualization of the device.

In the collagen-fiber composite tube, the fibers carry much of the hoop stress and other loadings imposed by the vessel. This relieves the loading on the collagen and significantly increases the burst strength and fatigue properties of the tube. In addition, this makes the tube more effective in hydraulically isolating the vessel and as a result prevents the formation or worsening of aneurysms. This would be particularly beneficial in thinned weakened vessel walls resulting from de-bulking interventions or from medial thinning that has been seen to accompany stent placement. Another benefit of the fiber reinforcement is the increase in resistance to radially inward loading, especially if the loading is very focused. Finally, fiber reinforcement may also impart some longitudinal stiffness to the stent-graft. This allows the stent-graft to maintain its strength and prevent it from kinking or sagging into the lumen.

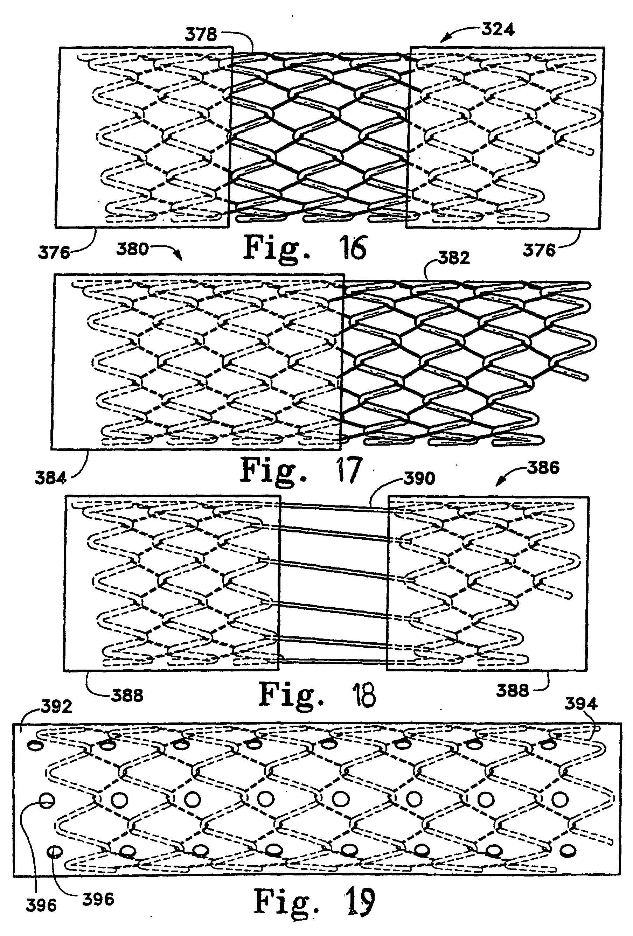

In some instances it is desirable to produce a stent-graft having a non-continuous graft member. For instance, Figure 15 shows a situation in which a stent-graft (370) having a continuous graft layer has been deployed in an artery over a side branch (372) thereby blocking perfusion to that branch (372). In some cases, a significant amount of tissue may be compromised as a result. Bare stents of the same configuration as the stent in stent-graft (370) would be adequate to allow 16 flow of blood into that side branch (372). Figures 16, 17, and 18 depict combination scent-grafts which have non-continuous graft members. In Figure 16 is found a stent-graft (374) having two separate graft sections (376) with a bare stent section (378) in the center. The bare stent section (378) allows blood flow through the stent mesh for side branches as seen in Figure 15. A further variation is seen in Figure 17. The combination stent-graft (380) with a bare stent end (382) and a single end graft (384). Figure 18 shows still another variation of the combination stent-graft (386) in which two short stent sections (388) associated with a graft material are separated by a series of links (390). The central link section is sufficient to allow flow of blood (or other fluids) through that area.

Another variation in which the graft layer (392) is discontinuous over the stent (394) is shown in Figure 19. In this instance the discontinuity is formed through the presence of discrete holes (396) through the graft layer. When used in a blood vessel, the stent-graft with holes will allow endothelial cells on the outside of the vessel to grow onto the inside of the stent-graft. Conventional vascular grafts only allow endothelial cells in the vessel to grow on the inner or flowing surface to grow from the ends of the graft.

### Deployment of the Invention

When a stent-graft having torsion members is folded, crushed, or otherwise collapsed, mechanical energy is stored as a twist in those torsion members. In this loaded state, the torsion members have a torque exerted about them and consequently have a tendency to untwist. Collectively, the torque exerted by the torsion members as folded down to a reduced diameter must be restrained from springing open. The stent typically has at least one torsion member per fold to take advantage of the invention. The stent-graft is folded along its longitudinal axis and restrained from springing open. The stent-graft is then deployed by removing the restraining mechanism, thus allowing the torsion members to spring open against the vessel wall.

The attending surgeon will choose a stent or stent-graft having an appropriate diameter. However, inventive devices of this type are typically selected having an expanded diameter of up to about 10% greater than the diameter of the lumen to be the site of the stent deployment.

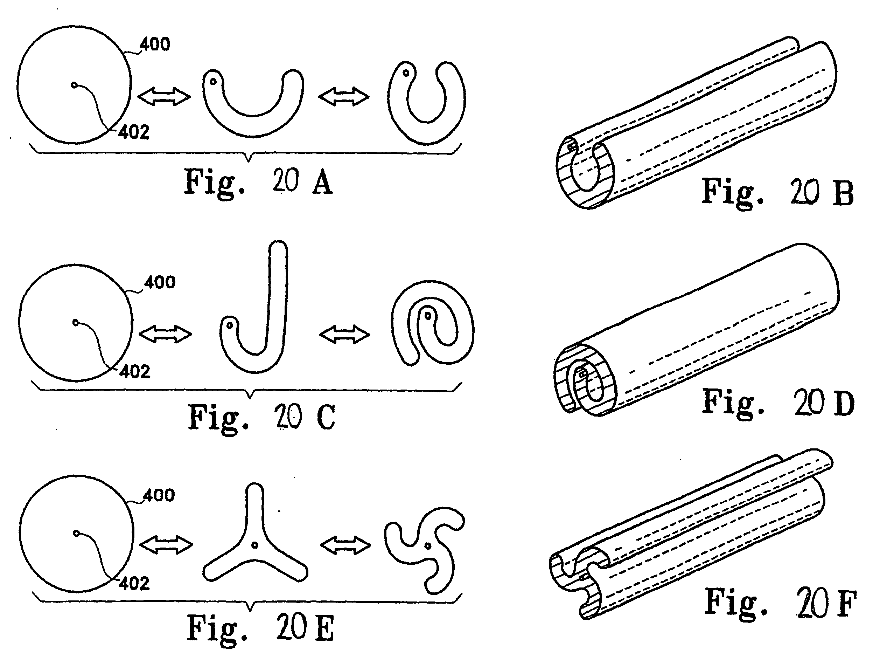

Figure 20A shows a sequence of folding the tubular device (400) of this invention about a guidewire (402) into a loose C-shaped configuration. Figure 20B shows a front quarter view of the resulting folded stent or stent-graft.

Figure 20C shows a sequence of folding the device (400) of this invention about a guidewire (402) into a rolled configuration. Figure 20D shows a front quarter view of the resulting folded stent or stent-graft.

Figure 20E shows a sequence of folding the device (400) of this invention about a guidewire (402) into a triple lobed configuration. Figure 20F shows a front quarter view of the resulting folded stent or stent-graft.

The stent-graft may be tracked through the vasculature (or other bodily lumen) to the intended deployment site and then unfolded against the vessel lumen. The graft tube component of the stent-graft is limp, flexible, and thus easy to fold. Folding of the stent structure in the manner discussed above allows it to return to a circular, open configuration.

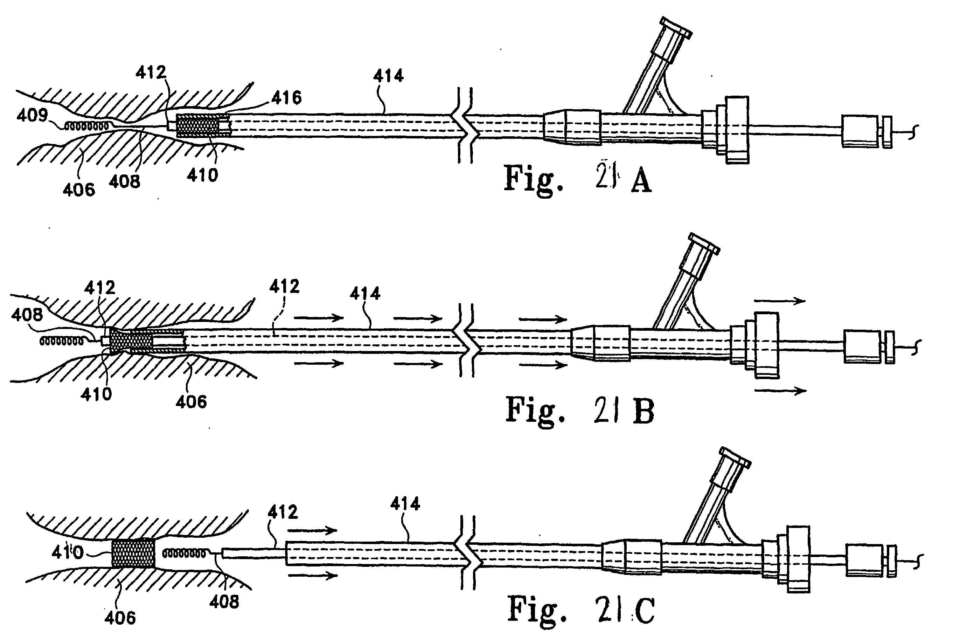

Figures 21A-21C show one desired way to place the devices of the present invention and allow them to self-expand. Figure 21A shows a target site (406) having, e.g., a narrowed vessel lumen. A guidewire (408) having a guide tip (409) has been directed to the site using known techniques. The stent-graft (410) is mounted on tubing (412) inside outer sliding sheath (416) after having folded in the manner discussed above. The outer sliding sheath (416) binds the compressed stent-graft (410) in place until released.

Figure 21B shows placement of the stent-graft (410) at the selected site (406) by sliding the stent-graft (410) over the guidewire (408) all together with the guidewire tubing (412) and the outer sliding sheath (414). The stent-graft (410) is deployed by holding the guidewire tubing (412) in a stationary position while withdrawing the outer sliding sheath (414). The stent-graft (410) can be seen in Figure 21B as partially deployed.

Figure 21C shows the stent-graft (410) fully deployed after the guidewire tubing (412) and the outer sliding sheath (414) have been fully retracted.

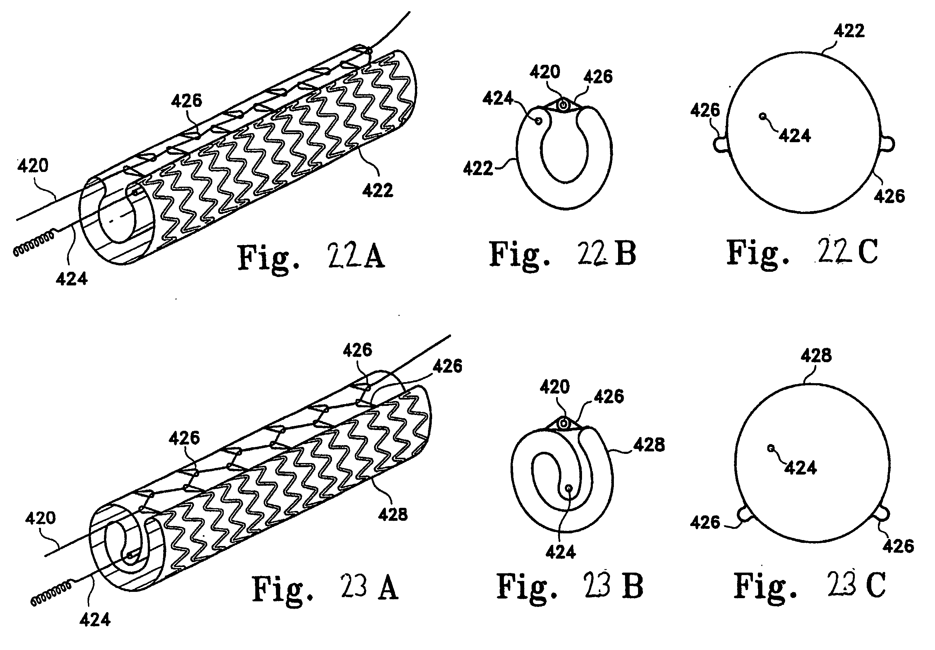

Figures 22A-C, 23 A-C and 24 A-C show an inventive variation of deploying a stent or stent-graft made according to this invention. These methods involve the use of a control line or tether line (420) which maintains the stent or stent-graft in a folded configuration until release.

Figure 22A is a front-quarter view of the stent (422) or stent-graft which has been folded as shown in the Figures discussed above. The stent (422) is folded about guidewire (424) so that, when deployed, the guidewire (424) is within the stent (422). Central to the variation shown here is the tether wire (420) which is passed through loops (426) associated with the various helices as they wind about the stent (422). The loops (426) may be formed from the flexible link (124 in Figures 2 or 3) or may be simply an alternating weave through appropriate apexes of the undulating helix, e.g., (104 in Figure 3) or may be loops specifically installed for the purpose shown here. It should be clear that the tether wire (426) is so placed that when it is removed by sliding it axially along the stent (422) and out of the loops (426), that the stent (422) unfolds into a generally cylindrical shape within the body lumen.

Figure 22B shows an end-view of a folded stent (422) or stent-graft having a guidewire (424) within the inner surface of the stent (422) and with the tether wire (420) within the loops (426). The end view of the folded stent (422) shows it to be folded into a form which is generally C-shaped. When expanded by removal of the tether wire (420), the stent (422) in Figure 22B assumes the form shown in end view in Figure 22B. There may be seen the guidewire (424) within the lumen of the stent (422) and the loops (426) which were formerly in a generally linear relationship having a tether wire passing through them.

Figure 23A shows a folded stent (428) (or stent-graft) in front quarter view which is similar in configuration to the stent (422) shown in Figure 22A except that the stent (428) is rolled somewhat tighter than the previously discussed stent. The guidewire (424) is also inside the stent (428) rather than outside of it. Loops (426) from generally opposing sides of the stent (428) are folded into an approximate line so that the tether wire may pass through the aligned loops (426). Figure 23B shows an end view of the stent (428), and in particular, emphasizes the tighter fold of the stent (428). When expanded by removal of the tether wire (420), the stent (428) in Figure 23B assumes the form shown in Figure 20C. In Figure 20C may be seen the guidewire (424) within the lumen of the stent (428) and the loops (426) which were formerly in a generally linear relationship having a tether wire passing through them.

Figures 24A-C show a schematic procedure for deploying the stent (430) (or stent-graft) using a percutaneous catheter assembly (432).

In Figure 24A may be seen a percutaneous catheter assembly (432) which has been inserted to a selected site (434) within a body lumen. The stent (430) is folded about the guidewire and guidewire tube (436) held axially in place prior to deployment by distal barrier (438) and proximal barrier (440). The distal barrier (438) and proximal barrier (440) typically are affixed to the guidewire tube (436). The tether wire (420) is shown extending through loops (426) proximally through the catheter assembly's (432) outer jacket (442) through to outside the body.

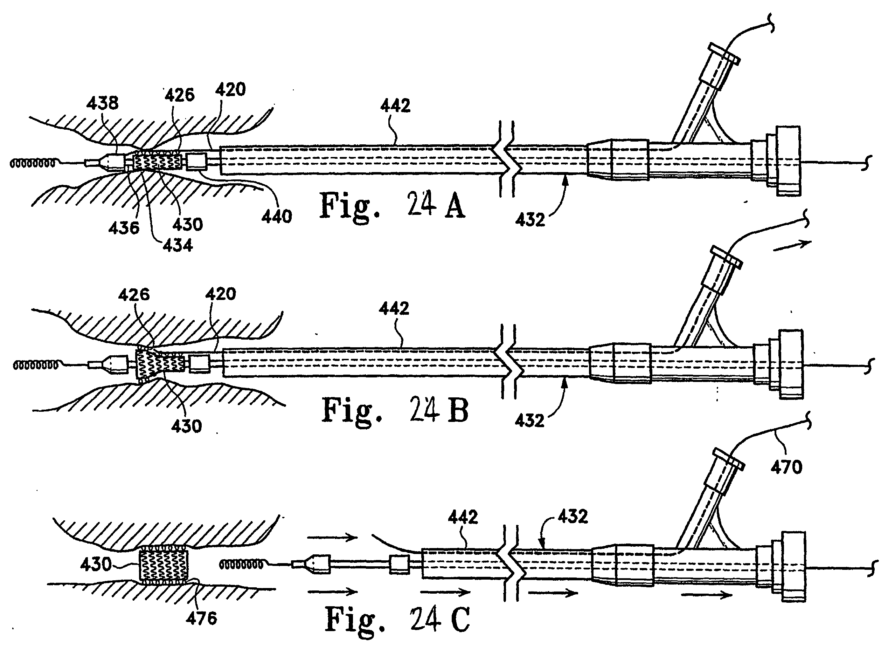

Figure 24B shows the removal of the tether wire (420) from a portion of the loops (426) to partially expand the stent (430) onto the selected site (434).

Figure 24C shows the final removal of the tether wire (420) from the loops (426) and the retraction of the catheter assembly (432) from the interior of the stent (430). The stent (430) is shown as fully expanded.

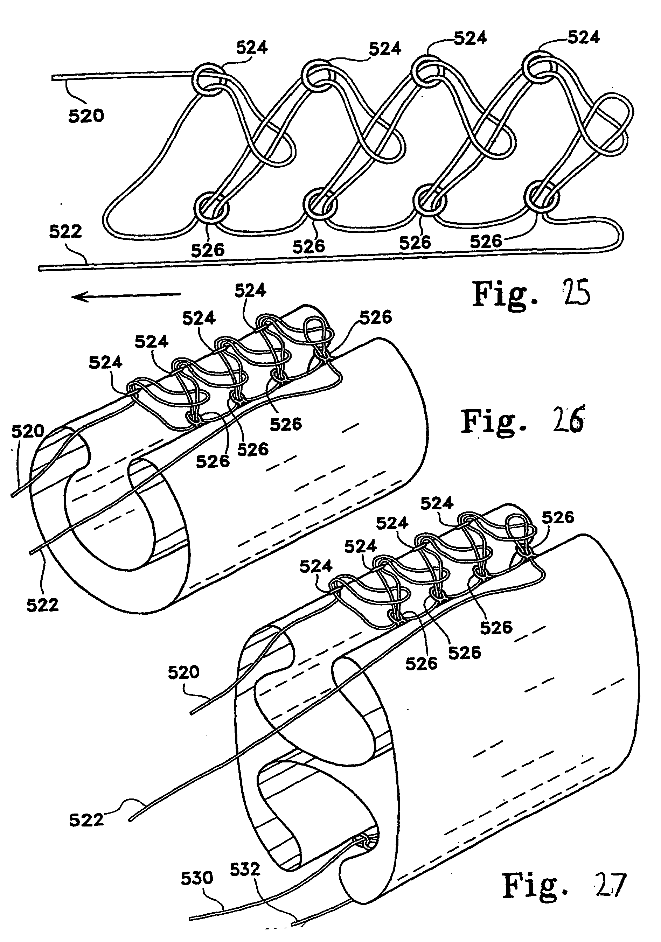

Figure 25 shows a close-up of a stent fold line having the familiar herringbone pattern of the "sack knot" used to close the fold in the stent. This knot is the one used to hold, e.g.; burlap sacks of feed grain closed prior to use and yet allow ease of opening when the sack is to be opened. In this variation, the slip line has a fixed end (520) and a release end (522). loops of the slip line pass through the eyelets (524) on the side of the stent fold associated with the fixed end (520) and are held in place by eyelets (526) on the side of the stent fold associated with the release end (522). The fixed end (520) is not typically tied to the stent so to allow removal of the slip line after deployment. The eyelets (524 and 526) are desirable but optional. The eyelets (524 and 526) may be wire or polymeric thread or the like tied to the stent structure at the edge of the stent fold. If so desired, the loops may be dispensed with and the slip line woven directly into the stent structure. The self-expanding stent may be deployed by pulling axially on release end (522) as shown by the arrow in the drawing.

Figures 26 and 27 show front quarter, views of folded stents using the knot shown in Figure 25. Figure 26 shows the use of a single stent fold similar in configuration to those described above. As was shown in Figure 25, the fixed end (520) portion of the slip line is associated with a row of eyelets (524) which are tied or otherwise fixed to the stent. The release end (522) is associated with the other row of eyelets (526).

Figure 24 depicts the use of multiple stent folds each having a fixed end (520 & 530) and a release end (522 & 532) on their respective slip lines.

The variations of the invention shown in Figures 25-27 may be introduced in to the body using the procedures outlined above with relation to Figures 21-24.

Although we generally discuss the deployment of the stent or stent-graft using a catheter, often deployed percutaneously, it should be apparent that the procedure and the folded stent or stent-graft are not so limited. The folded stent or stent-graft may also be deployed through artificial or natural body openings with a sheath or endoscopic delivery device perhaps without a guidewire. Similarly, the stent or stent graft may be delivered manually during a surgical procedure.

## Claims

1. A device comprising:
a tubular self-expanding stent (122) defined by a helically wrapped undulating member (100) containing multiple turns about a common longitudinal axis, said undulating member containing a plurality of undulations, each undulation having an apex, (104), two arms (106) and an amplitude; and
a flexible linkage (124) weaved to extend through the undulations of adjacent turns from one turn of the helix to the other and maintaining an undulation of one helical turn in phase with an undulation in an adjacent helical turn;
**characterised in that** said undulations are open or unconfined at their apex such that the flexible linkage (124) may move unconstrainedly away from each apex, and said apex (104) does not have any means **in that** apex that would tend to inhibit the movement of the flexible linkage (124) down between the arms of the undulations.

2. A device as claimed in claim 1, wherein said undulating member (100) is formed from sheet material.

3. A device as claimed in claim 2, wherein said undulating member (100) is formed from tubing.

4. A device as claimed in claim 1, wherein said undulating member (100) is formed from wire.

5. A device as claimed in any preceding claim, wherein said undulating member (100) comprises a nickel-titanium alloy.

6. A device as claimed in any of claims 1 to 4, wherein said undulating member (100) comprises a superelastic alloy.

7. A device as claimed in any preceding claim, wherein adjacent undulations in said undulating member (100) have different amplitudes.

8. A device as claimed in any of claims 1 to 6, wherein adjacent undulations in said undulating member (100) have substantially the same amplitude.

9. A device as claimed in any preceding claim, wherein said undulations are substantially sinusoidal, U-shaped or V-shaped.

10. A device as claimed in any preceding claim, further including a coaxially positioned, polymeric tubular graft (134) coupled to said stent (122).

11. A device as claimed in claim 10, wherein said polymeric tubular graft (134) is positioned within said tubular stent (122).

12. A device as claimed in claim 10 or 11, wherein said polymeric graft (134) comprises a fluoropolymer.

13. A device as claimed in claim 12, wherein said fluoropolymer comprises expanded polytetrafluoroethylene.

14. A device as claimed in any preceding claim, wherein said stent (122) has at least one flared end (142).

15. A device as claimed in any preceding claim, wherein the apexes (104) of one helical turn are longitudinally spaced from the apexes (104) in adjacent helical turns.

## Patentansprüche

1. Vorrichtung, die aufweist:
einen schlauchartigen selbstausdehnenden Stent (122), der durch ein schraubenförmig gewickeltes wellenartiges Element (100) definiert wird, das mehrfache Windungen um eine gemeinsame Längsachse einschließt, wobei das wellenartige Element eine Vielzahl von Wellen enthält, wobei jede Welle einen Scheitel (104), zwei Schenkel (106) und eine Amplitude aufweist; und
eine flexible Verbindung (124), die so eingefügt ist, daß sie sich durch die Wellen der angrenzenden Windungen von einer Windung der Schraube zur anderen erstreckt und eine Welle einer schraubenförmigen Windung in Phase mit einer Welle in einer angrenzenden schraubenförmigen Windung hält;
**dadurch gekennzeichnet, daß** die Wellen an ihrem Scheitel offen oder nicht begrenzt sind, so daß sich die flexible Verbindung (124) unbehindert von jedem Scheitel weg bewegen kann, und der Scheitel (104) nicht irgendein Hilfsmittel in jenem Scheitel aufweist, das dazu neigen würde, die Bewegung der flexiblen Verbindung (124) nach unten zwischen die Schenkel der Wellen zu behindern.

2. Vorrichtung nach Anspruch 1, bei der das wellenartige Element (100) aus Folienmaterial gebildet wird.

3. Vorrichtung nach Anspruch 2, bei der das wellenartige Element (100) aus Schlauch gebildet wird.

4. Vorrichtung nach Anspruch 1, bei der das wellenartige Element (100) aus Draht gebildet wird.

5. Vorrichtung nach vorhergehenden Ansprüchen, bei der das wellenartige Element (100) eine Nickel-Titan-Legierung aufweist.

6. Vorrichtung nach einem der Ansprüche 1 bis 4, bei der das wellenartige Element (100) eine superelastische Legierung aufweist.

7. Vorrichtung nach vorhergehenden Ansprüchen, bei der angrenzende Wellen im wellenartigen Element (100) unterschiedliche Amplituden aufweisen.

8. Vorrichtung nach einem der Ansprüche 1 bis 6, bei der angrenzende Wellen im wellenartigen Element (100) im wesentlichen die gleiche Amplitude aufweisen.

9. Vorrichtung nach vorhergehenden Ansprüchen, bei der die Wellen im wesentlichen sinusförmig, U-förmig oder V-förmig sind.

10. Vorrichtung nach vorhergehenden Ansprüchen, die außerdem ein koaxial angeordnetes polymeres schlauchartiges Transplantat (134) umfaßt, das mit dem Stent (122) gekoppelt ist.

11. Vorrichtung nach Anspruch 10, bei der das polymere schlauchartige Transplantat (134) innerhalb des schlauchartigen Stents (122) angeordnet ist.

12. Vorrichtung nach Anspruch 10 oder 11, bei der das polymere Transplantat (134) ein Fluorpolymer aufweist.

13. Vorrichtung nach Anspruch 12, bei der das Fluorpolymer aufgeschäumtes Polytetrafluorethylen aufweist.

14. Vorrichtung nach vorhergehenden Ansprüchen, bei der der Stent (122) mindestens ein aufgeweitetes Ende (142) aufweist.

15. Vorrichtung nach vorhergehenden Ansprüchen, bei der die Scheitel (104) einer schraubenförmigen Windung in Längsrichtung von den Scheiteln (104) in angrenzenden schraubenförmigen Windungen beabstandet sind.

## Revendications

1. Dispositif, comprenant
un stent tubulaire à auto-dilatation (122) défini par un élément ondulatoire à enroulement hélicoïdal (100) contenant des spires multiples autour d'un axe longitudinal commun, ledit élément ondulatoire contenant plusieurs ondulations, chaque ondulation comportant un apex (104), deux bras (106) et une amplitude; et
un élément de liaison flexible (124) tissé en vue d'une extension à travers les ondulations de spires adjacentes, d'une spire de l'hélice vers l'autre, et maintenant une ondulation d'une spire hélicoïdale en phase avec une ondulation d'une spire hélicoïdale adjacente;
**caractérisé en ce que** lesdites ondulations sont ouvertes ou non confinées au niveau de leur apex, de sorte que l'élément de liaison flexible (124) peut se déplacer sans contrainte à l'écart de chaque apex, ledit apex (104) ne comportant aucun moyen dans cet apex tendant à inhiber le déplacement de l'élément de liaison flexible (124) vers le bas entre les bras des ondulations.

2. Dispositif selon la revendication 1, dans lequel ledit élément ondulatoire (100) est formé à partir d'un matériau en feuille.

3. Dispositif selon la revendication 2, dans lequel ledit élément ondulatoire (100) est formé par des tubes.

4. Dispositif selon la revendication 1, dans lequel ledit élément ondulatoire (100) est formé à partir de fil métallique.

5. Dispositif selon l'une quelconque des revendications précédentes, dans lequel ledit élément ondulatoire (100) comprend un alliage de nickel-titane.

6. Dispositif selon l'une quelconque des revendications 1 à 4, dans lequel ledit élément ondulatoire (100) comprend un alliage superélastique.

7. Dispositif selon l'une quelconque des revendications précédentes, dans lequel les ondulations adjacentes dans ledit élément ondulatoire (100) ont des amplitudes différentes.

8. Dispositif selon l'une quelconque des revendications 1 à 6, dans lequel les ondulations adjacentes dans ledit élément ondulatoire (100) ont pratiquement la même amplitude.

9. Dispositif selon l'une quelconque des revendications précédentes, dans lequel lesdites ondulations sont pratiquement sinusoïdales, en forme de U ou de V.

10. Dispositif selon l'une quelconque des revendications précédentes, englobant en outre une greffe tubulaire polymère à positionnement coaxial (134) couplée audit stent (122).

11. Dispositif selon la revendication 10, dans lequel ladite greffe tubulaire polymère (134) est positionnée dans ledit stent tubulaire (122).

12. Dispositif selon les revendications 10 ou 11, dans lequel ladite greffe polymère (134) comprend un polymère fluoré.

13. Dispositif selon la revendication 12, dans lequel ledit polymère fluoré comprend du polytétrafluoréthylène expansé.

14. Dispositif selon l'une quelconque des revendications précédentes, dans lequel ledit stent (122) comporte au moins une extrémité évasée (142).

15. Dispositif selon l'une quelconque des revendications précédentes, dans lequel les apex (104) d'une spire hélicoïdale sont espacés longitudinalement des apex (104) des spires hélicoïdales adjacentes.
